# FASCICULE DE BREVET EUROPEEN

(11) **EP 1 621 547 B1**
(45) Date de publication et mention de la délivrance du brevet: **17.02.2010**
(21) Numéro de dépôt: 05016651.1
(22) Date de dépôt: 12.06.1996
(51) Int. Cl.: C07K 14/445, A61K 39/015

(54) **Conjugués d' un polypeptide de stade pre-erythrocytaire du paludisme et d'un résidu lipidique**
Gegen Malaria polypeptidische Molekulen von vorerythrozytären Stadium und lipiden Konjugate
Conjugates of malarial pre-erythrocytic stage polypeptide molecules and lipids

(30) Priorité: 13.06.1995 FR 9507007
(43) Date de publication de la demande: 01.02.2006
(62) Demande divisionnaire de: 96922107.6
(73) Titulaire: INSTITUT PASTEUR, 75724 Paris Cedex 15 (FR)
(72) Inventeur: Druilhe, Pierre, 75015 Paris (FR); Daubersies, Pierre, 59552 Lambres lez Douai (FR)
(74) Mandataire: Desaix, Anne

(56) Documents cités:
- WO-A-92/13884
- WO-A-94/09140
- FR-A- 2 679 909
- MARTINON FREDERIC ET AL: "Immunization of mice with lipopeptides bypasses the prerequisite for adjuvant: Immune response of BALB/c mice to human immunodeficiency virus envelope glycoprotein" JOURNAL OF IMMUNOLOGY, vol. 149, no. 10, 1992, pages 3416-3422, XP002354318 ISSN: 0022-1767
- ROUAIX F ET AL: "Effect of a lipopeptidic formulation on macrophage activation and peptide presentation to T cells" VACCINE, BUTTERWORTH SCIENTIFIC. GUILDFORD, GB, vol. 12, no. 13, 1994, pages 1209-1214, XP002082968 ISSN: 0264-410X
- FIDOCK D A ET AL: "Cloning and characterization of a novel Plasmodium falciparum sporozoite surface antigen, STARP." MOLECULAR AND BIOCHEMICAL PARASITOLOGY 64 (2). 1994. 219-232, 1994, XP000611837
- DAUBERSIES P. ET AL: 'Protection against Plasmodium falciparum malaria in chimpanzees by immunization with the conserved pre-erythrocytic liver-stage antigen 3', vol. 6, partie 11 Novembre 2000, NATURE MEDICINE, ISSN 1078-8956 pages 1258 - 1263
- BENMOHAMED L. ET AL: "Lipopeptide vaccines - yesterday, today and tomorrow" LANCET INFECTIOUS DISEASES, vol. 2, no. 7, July 2002 (2002-07), pages 425-431, XP004811155 US
- BENMOHAMED L. ET AL: "High immunogenicity in chimpanzees of peptides and lipopeptides from four new Plamodium falciparum pre-erythrocytic molecules" VACCINE, vol. 18, no. 25, June 2000 (2000-06), pages 2843-2855, XP004203575 BUTTERWORTH SCIENTIFIC. GUILDFORD, GB

### SEQUENCE LISTING

<110> INSTITUT PASTEUR
<120> MALARIAL PRE-ERYTHROCYTIC STAGE POLYPEPTIDE MOLECULES
<130> B277SAA - JAZ
<140> EP 96 922 107.6
   <141> 1996-06-12
<150> FR 95 07 007
   <151> 1995-06-13
<150> PCT/FR 96/00894
   <151> 1996-06-12
<160> 7
<170> PatentIn version 3.1
<210> SEQ ID No 1
   <211> 6152
   <212> DNA
   <213> NUCLEOTIDE
<400> 1 
atttatttat ttttattgtt ttatttcttt tttttcttta aattgtatat ttataaatat 60
tttaaaaagt tagaaaatga caaatagtaa ttacaaatca aataataaaa catataatga 120
aaataataat gaacaaataa ctaccatatt taatagaaca aatatgaatc cgataaaaaa 180
atgtcatatg agagaaaaaa taaataagta cttttttttg atcaaaattt tgacatgcac 240
cattttaata tgggctgtac aatatgataa taacgtaaga taaaaaacta aataataaat 300
ataaataaaa aaaaaaaaaa aaaaaaaaaa atcaactata tagtatgtat aatatatata 360
tatatatata tatatatata tatatatata tatttatttt tatttattta ttaatttttt 420
tttttttata ttatcttttt agtctgatat aaacaagagt tggaaaaaaa atacgtatgt 480
agataagaaa ttgaataaac tatttaacag aagtttagga gaatctcaag taaatggtga 540
attagctagt gaagaagtaa aggaaaaaat tcttgactta ttagaagaag gaaatacatt 600
aactgaaagt gtagatgata ataaaaattt agaagaagcc gaagatataa aggaaaatat 660
cttattaagt aatatagaag aaccaaaaga aaatattatt gacaatttat taaataatat 720
tggacaaaat tcagaaaaac aagaaagtgt atcagaaaat gtacaagtca gtgatgaact 780
ttttaatgaa ttattaaata gtgtagatgt taatggagaa gtaaaagaaa atattttgga 840
ggaaagtcaa gttaatgacg atatttttaa tagtttagta aaaagtgttc aacaagaaca 900
acaacacaat gttgaagaaa aagttgaaga aagtgtagaa gaaaatgacg aagaaagtgt 960
agaagaaaat gtagaagaaa atgtagaaga aaatgacgac ggaagtgtag cctcaagtgt 1020
tgaagaaagt atagcttcaa gtgttgatga aagtatagat tcaagtattg aagaaaatgt 1080
agctccaact gttgaagaaa tcgtagctcc aagtgttgta gaaagtgtgg ctccaagtgt 1140
tgaagaaagt gtagaagaaa atgttgaaga aagtgtagct gaaaatgttg aagaaagtgt 1200
agctgaaaat gttgaagaaa gtgtagctga aaatgttgaa gaaagtgtag ctgaaaatgt 1260
 
tgaagaaatc gtagctccaa ctgttgaaga aatcgtagct ccaactgttg aagaaattgt 1320
agctccaagt gttgtagaaa gtgtggctcc aagtgttgaa gaaagtgtag aagaaaatgt 1380
tgaagaaagt gtagctgaaa atgttgaaga aagtgtagct gaaaatgttg aagaaagtgt 1440
agctgaaaat gttgaagaaa gtgtagctga aaatgttgaa gaaagtgtag ctgaaaatgt 1500
tgaagaaatc gtagctccaa ctgttgaaga aatcgtagct ccaactgttg aagaaattgt 1560
agctccaagt gttgtagaaa gtgtggctcc aagtgttgaa gaaagtgtag aagaaaatgt 1620
tgaagaaagt gtagctgaaa atgttgaaga aagtgtagct gaaaatgttg aagaaagtgt 1680
agctgaaaat gttgaagaaa gtgtagctga aaatgttgaa gaaagtgtag ctgaaaatgt 1740
tgaagaaagt gtagctgaaa atgttgaaga aagtgtagct gaaaatgttg aagaaatcgt 1800
agctccaact gttgaagaaa tcgtagctcc aactgttgaa gaaattgtag ctccaagtgt 1860
tgtagaaagt gtggctccaa gtgttgaaga aagtgtagaa gaaaatgttg aagaaagtgt 1920
agctgaaaat gttgaagaaa gtgtagctga aaatgttgaa gaaagtgtag ctgaaaatgt 1980
tgaagaaagt gtagctgaaa atgttgaaga aatcgtagct ccaactgttg aagaaatcgt 2040
agctccaact gttgaagaaa ttgtagctcc aagtgttgta gaaagtgtgg ctccaagtgt 2100
tgaagaaagt gtagaagaaa atgttgaaga aagtgtagct gaaaatgttg aagaaagtgt 2160
agctgaaaat gttgaagaaa gtgtagctga aaatgttgaa gaaatcgtag ctccaactgt 2220
tgaagaaatc gtagctccaa ctgttgaaga aattgtagct ccaagtgttg tagaaagtgt 2280
ggctccaagt gttgaagaaa gtgtagaaga aaatgttgaa gaaagtgtag ctgaaaatgt 2340
tgaagaaagt gtagctgaaa atgttgaaga aagtgtagct gaaaatgttg aagaaagtgt 2400
agctgaaaat gttgaagaaa tcgtagctcc aactgttgaa gaaatcgtag ctccaactgt 2460
tgaagaaatt gtagctccaa gtgttgtaga aagtgtggct ccaagtgttg aagaaagtgt 2520
agaagaaaat gttgaagaaa gtgtagctga aaatgttgaa gaaagtgtag ctgaaaatgt 2580
tgaagaaagt gtagctgaaa atgttgaaga aagtgtagct ccaactgttg aagaaattgt 2640
agctccaagt gttgaagaaa gtgtagctcc aagtgttgaa gaaagtgttg ctgaaaacgt 2700
tgcaacaaat ttatcagaca atcttttaag taatttatta ggtggtatcg aaactgagga 2760
aataaaggac agtatattaa atgagataga agaagtaaaa gaaaatgtag tcaccacaat 2820
actagaaaac gtagaagaaa ctacagctga aagtgtaact acttttagta acatattaga 2880
ggagatacaa gaaaatacta ttactaatga tactatagag gaaaaattag aagaactcca 2940
cgaaaatgta ttaagtgccg ctttagaaaa tacccaaagt gaagaggaaa agaaagaagt 3000
aatagatgta attgaagaag taaaagaaga ggtcgctacc actttaatag aaactgtgga 3060
acaggcagaa gaaaagagcg caaatacaat tacggaaata tttgaaaatt tagaagaaaa 3120
tgcagtagaa agtaatgaaa atgttgcaga gaatttagag aaattaaacg aaactgtatt 3180
taatactgta ttagataaag tagaggaaac agtagaaatt agcggagaaa gtttagaaaa 3240
caatgaaatg gataaagcat tttttagtga aatatttgat aatgtaaaag gaatacaaga 3300
 
aaatttatta acaggtatgt ttcgaagtat agaaaccagt atagtaatcc aatcagaaga 3360
aaaggttgat ttgaatgaaa atgtggttag ttcgatttta gataatatag aaaatatgaa 3420
agaaggttta ttaaataaat tagaaaatat ttcaagtact gaaggtgttc aagaaactgt 3480
aactgaacat gtagaacaaa atgtatatgt ggatgttgat gttcctgcta tgaaagatca 3540
atttttagga atattaaatg aggcaggagg gttgaaagaa atgtttttta atttggaaga 3600
tgtatttaaa agtgaaagtg atgtaattac tgtagaagaa attaaggatg aaccggttca 3660
aaaagaggta gaaaaagaaa ctgttagtat tattgaagaa atggaagaaa atattgtaga 3720
tgtattagag gaagaaaaag aagatttaac agacaagatg atagatgcag tagaagaatc 3780
catagaaata tcttcagatt ctaaagaaga aactgaatct attaaagata aagaaaaaga 3840
tgtttcacta gttgttgaag aagttcaaga caatgatatg gatgaaagtg ttgagaaagt 3900
tttagaattg aaaaatatgg aagaggagtt aatgaaggat gctgttgaaa taaatgacat 3960
tactagcaaa cttattgaag aaactcaaga gttaaatgaa gtagaagcag atttaataaa 4020
agatatggaa aaattaaaag aattagaaaa agcattatca gaagattcta aagaaataat 4080
agatgcaaaa gatgatacat tagaaaaagt tattgaagag gaacatgata taacgacgac 4140
gttggatgaa gttgtagaat taaaagatgt cgaagaagac aagatcgaaa aagtatctga 4200
tttaaaagat cttgaagaag atatattaaa agaagtaaaa gaaatcaaag aacttgaaag 4260
tgaaatttta gaagattata aagaattaaa aactattgaa acagatattt tagaagagaa 4320
aaaagaaata gaaaaagatc attttgaaaa attcgaagaa gaagctgaag aaataaaaga 4380
tcttgaagca gatatattaa aagaagtatc ttcattagaa gttgaagaag aaaaaaaatt 4440
agaagaagta cacgaattaa aagaagaggt agaacatata ataagtggtg atgcgcatat 4500
aaaaggtttg gaagaagatg atttagaaga agtagatgat ttaaaaggaa gtatattaga 4560
catgttaaag ggagatatgg aattagggga tatggataag gaaagtttag aagatgtaac 4620
aacaaaactt ggagaaagag ttgaatcctt aaaagatgtt ttatctagtg cattaggcat 4680
ggatgaagaa caaatgaaaa caagaaaaaa agctcaaaga cctaagttgg aagaagtatt 4740
attaaaagaa gaggttaaag aagaaccaaa gaaaaaaata acaaaaaaga aagtaaggtt 4800
tgatattaag gataaggaac caaaagatga aatagtagaa gttgaaatga aagatgaaga 4860
tatagaagaa gatgtagaag aagatataga agaagatata gaagaagata aagttgaaga 4920
tatagatgaa gatatagatg aagatatagg tgaagacaaa gatgaagtta tagatttaat 4980
agtccaaaaa gagaaacgca ttgaaaaggt taaagcgaaa aagaaaaaat tagaaaaaaa 5040
agttgaagaa ggtgttagtg gtcttaaaaa acacgtagac gaagtaatga aatatgttca 5100
aaaaattgat aaagaagttg ataaagaagt atctaaagct ttagaatcaa aaaatgatgt 5160
tactaatgtt ttaaaacaaa atcaagattt ttttagtaaa gttaaaaact tcgtaaaaaa 5220
atataaagta tttgctgcac cattcatatc tgccgttgca gcatttgcat catatgtagt 5280
tgggttcttt acattttctt tattttcatc atgtgtaaca atagcttctt caacttactt 5340
 
attatcaaaa gttgacaaaa ctataaataa aaataaggag agaccgtttt attcatttgt 5400

atttgatatc tttaagaatt taaaacatta tttacaacaa atgaaagaaa aatttagtaa 5460

agaaaaaaat aataatgtaa tagaagtaac aaacaaagct gagaaaaaag gtaatgtaca 5520

ggtaacaaat aaaaccgaga aaacaactaa agttgataaa aataataaag taccgaaaaa 5580

aagaagaacg caaaaatcaa aataaaaaat tgcagaagag tgaaatgatt ggagcgaaca 5640

ataaaattaa tcgataaaaa atataaaaat gtatatatta tgtaaatata tataaataaa 5700

taaataaata catacatata tatatatata tatatgtatc tttttacaaa attttaaaat 5760

tttaaaattt atatatatta atatttatat ttttccatat ataattttat tttcaatatt 5820

ttatttttaa ttataaatgt tttttacaga gtttatgttt tttaattaat atatagattt 5880

ctgtaagaaa ctgtatatta ttcatacgat atatgtaata ttaattattt gtgttttatt 5940

aaaatttata ttatataata tatatatata tatatatgta tatatattag aagataaaaa 6000

tttagcttat tttgcttgtt atgcaataag cttttttttt tttttttttt tttttttttc 6060

atataaacga tgtttaattt ttaattttta atattttata taaaatattt ttcctaaaaa 6120

aaaaaaaaat taaaaaaaac ttatatttcg aa 6152
<210> SEQ ID No 2
   <211> 5361
   <212> DNA
   <213> NUCLEOTIDE
<220>
   <221> CDS
   <222> (1)..(5361)
   <223>
<400> 2 
atg aca aat agt aat tac aaa tca aat aat aaa aca tat aat gaa aat 48
Met Thr Asn Ser Asn Tyr Lys Ser Asn Asn Lys Thr Tyr Asn Glu Asn
1               5                   10                  15

aat aat gaa caa ata act acc ata ttt aat aga aca aat atg aat ccg 96
Asn Asn Glu Gln Ile Thr Thr Ile Phe Asn Arg Thr Asn Met Asn Pro
            20                  25                  30

ata aaa aaa tgt cat atg aga gaa aaa ata aat aag tac ttt ttt ttg 144
Ile Lys Lys Cys His Met Arg Glu Lys Ile Asn Lys Tyr Phe Phe Leu
        35                  40                  45

atc aaa att ttg aca tgc acc att tta ata tgg gct gta caa tat gat 192
Ile Lys Ile Leu Thr Cys Thr Ile Leu Ile Trp Ala Val Gln Tyr Asp
    50                  55                  60

aat aac tct gat ata aac aag agt tgg aaa aaa aat acg tat gta gat 240
Asn Asn Ser Asp Ile Asn Lys Ser Trp Lys Lys Asn Thr Tyr Val Asp
65                  70                  75                  80

aag aaa ttg aat aaa cta ttt aac aga agt tta gga gaa tct caa gta 288
Lys Lys Leu Asn Lys Leu Phe Asn Arg Ser Leu Gly Glu Ser Gln Val
                85                  90                  95

aat ggt gaa tta gct agt gaa gaa gta aag gaa aaa att ctt gac tta 336
Asn Gly Glu Leu Ala Ser Glu Glu Val Lys Glu Lys Ile Leu Asp Leu
            100                 105                 110
 
tta gaa gaa gga aat aca tta act gaa agt gta gat gat aat aaa aat 384
Leu Glu Glu Gly Asn Thr Leu Thr Glu Ser Val Asp Asp Asn Lys Asn
        115                 120                 125

tta gaa gaa gcc gaa gat ata aag gaa aat atc tta tta agt aat ata 432
Leu Glu Glu Ala Glu Asp Ile Lys Glu Asn Ile Leu Leu Ser Asn Ile
    130                 135                 140

gaa gaa cca aaa gaa aat att att gac aat tta tta aat aat att gga 480
Glu Glu Pro Lys Glu Asn Ile Ile Asp Asn Leu Leu Asn Asn Ile Gly
145                 150                 155                 160

caa aat tca gaa aaa caa gaa agt gta tca gaa aat gta caa gtc agt 528
Gln Asn Ser Glu Lys Gln Glu Ser Val Ser Glu Asn Val Gln Val Ser
                165                 170                 175

gat gaa ctt ttt aat gaa tta tta aat agt gta gat gtt aat gga gaa 576
Asp Glu Leu Phe Asn Glu Leu Leu Asn Ser Val Asp Val Asn Gly Glu
            180                 185                 190

gta aaa gaa aat att ttg gag gaa agt caa gtt aat gac gat att ttt 624
Val Lys Glu Asn Ile Leu Glu Glu Ser Gln Val Asn Asp Asp Ile Phe
        195                 200                 205

aat agt tta gta aaa agt gtt caa caa gaa caa caa cac aat gtt gaa 672
Asn Ser Leu Val Lys Ser Val Gln Gln Glu Gln Gln His Asn Val Glu
    210                 215                 220

gaa aaa gtt gaa gaa agt gta gaa gaa aat gac gaa gaa agt gta gaa 720
Glu Lys Val Glu Glu Ser Val Glu Glu Asn Asp Glu Glu Ser Val Glu
225                 230                 235                 240

gaa aat gta gaa gaa aat gta gaa gaa aat gac gac gga agt gta gcc 768
Glu Asn Val Glu Glu Asn Val Glu Glu Asn Asp Asp Gly Ser Val Ala
                245                 250                 255

tca agt gtt gaa gaa agt ata gct tca agt gtt gat gaa agt ata gat 816
Ser Ser Val Glu Glu Ser Ile Ala Ser Ser Val Asp Glu Ser Ile Asp
            260                 265                 270

tca agt att gaa gaa aat gta gct cca act gtt gaa gaa atc gta gct 864
Ser Ser Ile Glu Glu Asn Val Ala Pro Thr Val Glu Glu Ile Val Ala
        275                 280                 285

cca agt gtt gta gaa agt gtg gct cca agt gtt gaa gaa agt gta gaa 912
Pro Ser Val Val Glu Ser Val Ala Pro Ser Val Glu Glu Ser Val Glu
    290                 295                 300

gaa aat gtt gaa gaa agt gta gct gaa aat gtt gaa gaa agt gta gct 960
Glu Asn Val Glu Glu Ser Val Ala Glu Asn Val Glu Glu Ser Val Ala
305                 310                 315                 320

gaa aat gtt gaa gaa agt gta gct gaa aat gtt gaa gaa agt gta gct 1008
Glu Asn Val Glu Glu Ser Val Ala Glu Asn Val Glu Glu Ser Val Ala
                325                 330                 335

gaa aat gtt gaa gaa atc gta gct cca act gtt gaa gaa atc gta gct 1056
Glu Asn Val Glu Glu Ile Val Ala Pro Thr Val Glu Glu Ile Val Ala
            340                 345                 350

cca act gtt gaa gaa att gta gct cca agt gtt gta gaa agt gtg gct 1104
Pro Thr Val Glu Glu Ile Val Ala Pro Ser Val Val Glu Ser Val Ala
        355                 360                 365

cca agt gtt gaa gaa agt gta gaa gaa aat gtt gaa gaa agt gta gct 1152
Pro Ser Val Glu Glu Ser Val Glu Glu Asn Val Glu Glu Ser Val Ala
    370                 375                 380
 
gaa aat gtt gaa gaa agt gta gct gaa aat gtt gaa gaa agt gta gct 1200
Glu Asn Val Glu Glu Ser Val Ala Glu Asn Val Glu Glu Ser Val Ala
385                 390                 395                 400

gaa aat gtt gaa gaa agt gta gct gaa aat gtt gaa gaa agt gta gct 1248
Glu Asn Val Glu Glu Ser Val Ala Glu Asn Val Glu Glu Ser Val Ala
                405                 410                 415

gaa aat gtt gaa gaa atc gta gct cca act gtt gaa gaa atc gta gct 1296
Glu Asn Val Glu Glu Ile Val Ala Pro Thr Val Glu Glu Ile Val Ala
            420                 425                 430

cca act gtt gaa gaa att gta gct cca agt gtt gta gaa agt gtg gct 1344
Pro Thr Val Glu Glu Ile Val Ala Pro Ser Val Val Glu Ser Val Ala
        435                 440                 445

cca agt gtt gaa gaa agt gta gaa gaa aat gtt gaa gaa agt gta gct 1392
Pro Ser Val Glu Glu Ser Val Glu Glu Asn Val Glu Glu Ser Val Ala
    450                 455                 460

gaa aat gtt gaa gaa agt gta gct gaa aat gtt gaa gaa agt gta gct 1440
Glu Asn Val Glu Glu Ser Val Ala Glu Asn Val Glu Glu Ser Val Ala
465                 470                 475                 480

gaa aat gtt gaa gaa agt gta gct gaa aat gtt gaa gaa agt gta gct 1488
Glu Asn Val Glu Glu Ser Val Ala Glu Asn Val Glu Glu Ser Val Ala
                485                 490                 495

gaa aat gtt gaa gaa agt gta gct gaa aat gtt gaa gaa agt gta gct 1536
Glu Asn Val Glu Glu Ser Val Ala Glu Asn Val Glu Glu Ser Val Ala
            500                 505                 510

gaa aat gtt gaa gaa atc gta gct cca act gtt gaa gaa atc gta gct 1584
Glu Asn Val Glu Glu Ile Val Ala Pro Thr Val Glu Glu Ile Val Ala
        515                 520                 525

cca act gtt gaa gaa att gta gct cca agt gtt gta gaa agt gtg gct 1632
Pro Thr Val Glu Glu Ile Val Ala Pro Ser Val Val Glu Ser Val Ala
    530                 535                 540

cca agt gtt gaa gaa agt gta gaa gaa aat gtt gaa gaa agt gta gct 1680
Pro Ser Val Glu Glu Ser Val Glu Glu Asn Val Glu Glu Ser Val Ala
545                 550                 555                 560

gaa aat gtt gaa gaa agt gta gct gaa aat gtt gaa gaa agt gta gct 1728
Glu Asn Val Glu Glu Ser Val Ala Glu Asn Val Glu Glu Ser Val Ala
                565                 570                 575

gaa aat gtt gaa gaa agt gta gct gaa aat gtt gaa gaa atc gta gct 1776
Glu Asn Val Glu Glu Ser Val Ala Glu Asn Val Glu Glu Ile Val Ala
            580                 585                 590

cca act gtt gaa gaa atc gta gct cca act gtt gaa gaa att gta gct 1824
Pro Thr Val Glu Glu Ile Val Ala Pro Thr Val Glu Glu Ile Val Ala
        595                 600                 605

cca agt gtt gta gaa agt gtg gct cca agt gtt gaa gaa agt gta gaa 1872
Pro Ser Val Val Glu Ser Val Ala Pro Ser Val Glu Glu Ser Val Glu
    610                 615                 620

gaa aat gtt gaa gaa agt gta gct gaa aat gtt gaa gaa agt gta gct 1920
Glu Asn Val Glu Glu Ser Val Ala Glu Asn Val Glu Glu Ser Val Ala
625                 630                 635                 640

gaa aat gtt gaa gaa agt gta gct gaa aat gtt gaa gaa atc gta gct 1968
Glu Asn Val Glu Glu Ser Val Ala Glu Asn Val Glu Glu Ile Val Ala
                645                 650                 655
 
cca act gtt gaa gaa atc gta gct cca act gtt gaa gaa att gta gct 2016
Pro Thr Val Glu Glu Ile Val Ala Pro Thr Val Glu Glu Ile Val Ala
            660                 665                 670

cca agt gtt gta gaa agt gtg gct cca agt gtt gaa gaa agt gta gaa 2064
Pro Ser Val Val Glu Ser Val Ala Pro Ser Val Glu Glu Ser Val Glu
        675                 680                 685

gaa aat gtt gaa gaa agt gta gct gaa aat gtt gaa gaa agt gta gct 2112
Glu Asn Val Glu Glu Ser Val Ala Glu Asn Val Glu Glu Ser Val Ala
    690                 695                 700

gaa aat gtt gaa gaa agt gta gct gaa aat gtt gaa gaa agt gta gct 2160
Glu Asn Val Glu Glu Ser Val Ala Glu Asn Val Glu Glu Ser Val Ala
705                 710                 715                 720

gaa aat gtt gaa gaa atc gta gct cca act gtt gaa gaa atc gta gct 2208
Glu Asn Val Glu Glu Ile Val Ala Pro Thr Val Glu Glu Ile Val Ala
                725                 730                 735

cca act gtt gaa gaa att gta gct cca agt gtt gta gaa agt gtg gct 2256
Pro Thr Val Glu Glu Ile Val Ala Pro Ser Val Val Glu Ser Val Ala
            740                 745                 750

cca agt gtt gaa gaa agt gta gaa gaa aat gtt gaa gaa agt gta gct 2304
Pro Ser Val Glu Glu Ser Val Glu Glu Asn Val Glu Glu Ser Val Ala
        755                 760                 765

gaa aat gtt gaa gaa agt gta gct gaa aat gtt gaa gaa agt gta gct 2352
Glu Asn Val Glu Glu Ser Val Ala Glu Asn Val Glu Glu Ser Val Ala
    770                 775                 780

gaa aat gtt gaa gaa agt gta gct cca act gtt gaa gaa att gta gct 2400
Glu Asn Val Glu Glu Ser Val Ala Pro Thr Val Glu Glu Ile Val Ala
785                 790                 795                 800

cca agt gtt gaa gaa agt gta gct cca agt gtt gaa gaa agt gtt gct 2448
Pro Ser Val Glu Glu Ser Val Ala Pro Ser Val Glu Glu Ser Val Ala
                805                 810                 815

gaa aac gtt gca aca aat tta tca gac aat ctt tta agt aat tta tta 2496
Glu Asn Val Ala Thr Asn Leu Ser Asp Asn Leu Leu Ser Asn Leu Leu
            820                 825                 830

ggt ggt atc gaa act gag gaa ata aag gac agt ata tta aat gag ata 2544
Gly Gly Ile Glu Thr Glu Glu Ile Lys Asp Ser Ile Leu Asn Glu Ile
        835                 840                 845

gaa gaa gta aaa gaa aat gta gtc acc aca ata cta gaa aac gta gaa 2592
Glu Glu Val Lys Glu Asn Val Val Thr Thr Ile Leu Glu Asn Val Glu
    850                 855                 860

gaa act aca gct gaa agt gta act act ttt agt aac ata tta gag gag 2640
Glu Thr Thr Ala Glu Ser Val Thr Thr Phe Ser Asn Ile Leu Glu Glu
865                 870                 875                 880

ata caa gaa aat act att act aat gat act ata gag gaa aaa tta gaa 2688
Ile Gln Glu Asn Thr Ile Thr Asn Asp Thr Ile Glu Glu Lys Leu Glu
                885                 890                 895

gaa ctc cac gaa aat gta tta agt gcc gct tta gaa aat acc caa agt 2736
Glu Leu His Glu Asn Val Leu Ser Ala Ala Leu Glu Asn Thr Gln Ser
            900                 905                 910

gaa gag gaa aag aaa gaa gta ata gat gta att gaa gaa gta aaa gaa 2784
Glu Glu Glu Lys Lys Glu Val Ile Asp Val Ile Glu Glu Val Lys Glu
        915                 920                 925
 
gag gtc gct acc act tta ata gaa act gtg gaa cag gca gaa gaa aag 2832
Glu Val Ala Thr Thr Leu Ile Glu Thr Val Glu Gln Ala Glu Glu Lys
    930                 935                 940

agc gca aat aca att acg gaa ata ttt gaa aat tta gaa gaa aat gca 2880
Ser Ala Asn Thr Ile Thr Glu Ile Phe Glu Asn Leu Glu Glu Asn Ala
945                 950                 955                 960

gta gaa agt aat gaa aat gtt gca gag aat tta gag aaa tta aac gaa 2928
Val Glu Ser Asn Glu Asn Val Ala Glu Asn Leu Glu Lys Leu Asn Glu
                965                 970                 975

act gta ttt aat act gta tta gat aaa gta gag gaa aca gta gaa att 2976
Thr Val Phe Asn Thr Val Leu Asp Lys Val Glu Glu Thr Val Glu Ile
            980                 985                 990

agc gga gaa agt tta gaa aac aat gaa atg gat aaa gca ttt ttt agt 3024
Ser Gly Glu Ser Leu Glu Asn Asn Glu Met Asp Lys Ala Phe Phe Ser
        995                 1000                1005

gaa ata ttt gat aat gta aaa gga ata caa gaa aat tta tta aca 3069
Glu Ile Phe Asp Asn Val Lys Gly Ile Gln Glu Asn Leu Leu Thr
    1010                1015                1020

ggt atg ttt cga agt ata gaa acc agt ata gta atc caa tca gaa 3114
Gly Met Phe Arg Ser Ile Glu Thr Ser Ile Val Ile Gln Ser Glu
    1025                1030                1035

gaa aag gtt gat ttg aat gaa aat gtg gtt agt tcg att tta gat 3159
Glu Lys Val Asp Leu Asn Glu Asn Val Val Ser Ser Ile Leu Asp
    1040                1045                1050

aat ata gaa aat atg aaa gaa ggt tta tta aat aaa tta gaa aat 3204
Asn Ile Glu Asn Met Lys Glu Gly Leu Leu Asn Lys Leu Glu Asn
    1055                1060                1065

att tca agt act gaa ggt gtt caa gaa act gta act gaa cat gta 3249
Ile Ser Ser Thr Glu Gly Val Gln Glu Thr Val Thr Glu His Val
    1070                1075                1080

gaa caa aat gta tat gtg gat gtt gat gtt cct gct atg aaa gat 3294
Glu Gln Asn Val Tyr Val Asp Val Asp Val Pro Ala Met Lys Asp
    1085                1090                1095

caa ttt tta gga ata tta aat gag gca gga ggg ttg aaa gaa atg 3339
Gln Phe Leu Gly Ile Leu Asn Glu Ala Gly Gly Leu Lys Glu Met
    1100                1105                1110

ttt ttt aat ttg gaa gat gta ttt aaa agt gaa agt gat gta att 3384
Phe Phe Asn Leu Glu Asp Val Phe Lys Ser Glu Ser Asp Val Ile
    1115                1120                1125

act gta gaa gaa att aag gat gaa ccg gtt caa aaa gag gta gaa 3429
Thr Val Glu Glu Ile Lys Asp Glu Pro Val Gln Lys Glu Val Glu
    1130                1135                1140

aaa gaa act gtt agt att att gaa gaa atg gaa gaa aat att gta 3474
Lys Glu Thr Val Ser Ile Ile Glu Glu Met Glu Glu Asn Ile Val
    1145                1150                1155

gat gta tta gag gaa gaa aaa gaa gat tta aca gac aag atg ata 3519
Asp Val Leu Glu Glu Glu Lys Glu Asp Leu Thr Asp Lys Met Ile
    1160                1165                1170

gat gca gta gaa gaa tcc ata gaa ata tct tca gat tct aaa gaa 3564
Asp Ala Val Glu Glu Ser Ile Glu Ile Ser Ser Asp Ser Lys Glu
    1175                1180                1185
 
gaa act gaa tct att aaa gat aaa gaa aaa gat gtt tca cta gtt 3609
Glu Thr Glu Ser Ile Lys Asp Lys Glu Lys Asp Val Ser Leu Val
    1190                1195                1200

gtt gaa gaa gtt caa gac aat gat atg gat gaa agt gtt gag aaa 3654
Val Glu Glu Val Gln Asp Asn Asp Met Asp Glu Ser Val Glu Lys
    1205                1210                1215

gtt tta gaa ttg aaa aat atg gaa gag gag tta atg aag gat gct 3699
Val Leu Glu Leu Lys Asn Met Glu Glu Glu Leu Met Lys Asp Ala
    1220                1225                1230

gtt gaa ata aat gac att act agc aaa ctt att gaa gaa act caa 3744
Val Glu Ile Asn Asp Ile Thr Ser Lys Leu Ile Glu Glu Thr Gln
    1235                1240                1245

gag tta aat gaa gta gaa gca gat tta ata aaa gat atg gaa aaa 3789
Glu Leu Asn Glu Val Glu Ala Asp Leu Ile Lys Asp Met Glu Lys
    1250                1255                1260

tta aaa gaa tta gaa aaa gca tta tca gaa gat tct aaa gaa ata 3834
Leu Lys Glu Leu Glu Lys Ala Leu Ser Glu Asp Ser Lys Glu Ile
    1265                1270                1275

ata gat gca aaa gat gat aca tta gaa aaa gtt att gaa gag gaa 3879
Ile Asp Ala Lys Asp Asp Thr Leu Glu Lys Val Ile Glu Glu Glu
    1280                1285                1290

cat gat ata acg acg acg ttg gat gaa gtt gta gaa tta aaa gat 3924
His Asp Ile Thr Thr Thr Leu Asp Glu Val Val Glu Leu Lys Asp
    1295                1300                1305

gtc gaa gaa gac aag atc gaa aaa gta tct gat tta aaa gat ctt 3969
Val Glu Glu Asp Lys Ile Glu Lys Val Ser Asp Leu Lys Asp Leu
    1310                1315                1320

gaa gaa gat ata tta aaa gaa gta aaa gaa atc aaa gaa ctt gaa 4014
Glu Glu Asp Ile Leu Lys Glu Val Lys Glu Ile Lys Glu Leu Glu
    1325                1330                1335

agt gaa att tta gaa gat tat aaa gaa tta aaa act att gaa aca 4059
Ser Glu Ile Leu Glu Asp Tyr Lys Glu Leu Lys Thr Ile Glu Thr
    1340                1345                1350

gat att tta gaa gag aaa aaa gaa ata gaa aaa gat cat ttt gaa 4104
Asp Ile Leu Glu Glu Lys Lys Glu Ile Glu Lys Asp His Phe Glu
    1355                1360                1365

aaa ttc gaa gaa gaa gct gaa gaa ata aaa gat ctt gaa gca gat 4149
Lys Phe Glu Glu Glu Ala Glu Glu Ile Lys Asp Leu Glu Ala Asp
    1370                1375                1380

ata tta aaa gaa gta tct tca tta gaa gtt gaa gaa gaa aaa aaa 4194
Ile Leu Lys Glu Val Ser Ser Leu Glu Val Glu Glu Glu Lys Lys
    1385                1390                1395

tta gaa gaa gta cac gaa tta aaa gaa gag gta gaa cat ata ata 4239
Leu Glu Glu Val His Glu Leu Lys Glu Glu Val Glu His Ile Ile
    1400                1405                1410

agt ggt gat gcg cat ata aaa ggt ttg gaa gaa gat gat tta gaa 4284
Ser Gly Asp Ala His Ile Lys Gly Leu Glu Glu Asp Asp Leu Glu
    1415                1420                1425

gaa gta gat gat tta aaa gga agt ata tta gac atg tta aag gga 4329
Glu Val Asp Asp Leu Lys Gly Ser Ile Leu Asp Met Leu Lys Gly
    1430                1435                1440
 
gat atg gaa tta ggg gat atg gat aag gaa agt tta gaa gat gta 4374
Asp Met Glu Leu Gly Asp Met Asp Lys Glu Ser Leu Glu Asp Val
    1445                1450                1455

aca aca aaa ctt gga gaa aga gtt gaa tcc tta aaa gat gtt tta 4419
Thr Thr Lys Leu Gly Glu Arg Val Glu Ser Leu Lys Asp Val Leu
    1460                1465                1470

tct agt gca tta ggc atg gat gaa gaa caa atg aaa aca aga aaa 4464
Ser Ser Ala Leu Gly Met Asp Glu Glu Gln Met Lys Thr Arg Lys
    1475                1480                1485

aaa gct caa aga cct aag ttg gaa gaa gta tta tta aaa gaa gag 4509
Lys Ala Gln Arg Pro Lys Leu Glu Glu Val Leu Leu Lys Glu Glu
    1490                1495                1500

gtt aaa gaa gaa cca aag aaa aaa ata aca aaa aag aaa gta agg 4554
Val Lys Glu Glu Pro Lys Lys Lys Ile Thr Lys Lys Lys Val Arg
    1505                1510                1515

ttt gat att aag gat aag gaa cca aaa gat gaa ata gta gaa gtt 4599
Phe Asp Ile Lys Asp Lys Glu Pro Lys Asp Glu Ile Val Glu Val
    1520                1525                1530

gaa atg aaa gat gaa gat ata gaa gaa gat gta gaa gaa gat ata 4644
Glu Met Lys Asp Glu Asp Ile Glu Glu Asp Val Glu Glu Asp Ile
    1535                1540                1545

gaa gaa gat ata gaa gaa gat aaa gtt gaa gat ata gat gaa gat 4689
Glu Glu Asp Ile Glu Glu Asp Lys Val Glu Asp Ile Asp Glu Asp
    1550                1555                1560

ata gat gaa gat ata ggt gaa gac aaa gat gaa gtt ata gat tta 4734
Ile Asp Glu Asp Ile Gly Glu Asp Lys Asp Glu Val Ile Asp Leu
    1565                1570                1575

ata gtc caa aaa gag aaa cgc att gaa aag gtt aaa gcg aaa aag 4779
Ile Val Gln Lys Glu Lys Arg Ile Glu Lys Val Lys Ala Lys Lys
    1580                1585                1590

aaa aaa tta gaa aaa aaa gtt gaa gaa ggt gtt agt ggt ctt aaa 4824
Lys Lys Leu Glu Lys Lys Val Glu Glu Gly Val Ser Gly Leu Lys
    1595                1600                1605

aaa cac gta gac gaa gta atg aaa tat gtt caa aaa att gat aaa 4869
Lys His Val Asp Glu Val Met Lys Tyr Val Gln Lys Ile Asp Lys
    1610                1615                1620

gaa gtt gat aaa gaa gta tct aaa gct tta gaa tca aaa aat gat 4914
Glu Val Asp Lys Glu Val Ser Lys Ala Leu Glu Ser Lys Asn Asp
    1625                1630                1635

gtt act aat gtt tta aaa caa aat caa gat ttt ttt agt aaa gtt 4959
Val Thr Asn Val Leu Lys Gln Asn Gln Asp Phe Phe Ser Lys Val
    1640                1645                1650

aaa aac ttc gta aaa aaa tat aaa gta ttt gct gca cca ttc ata 5004
Lys Asn Phe Val Lys Lys Tyr Lys Val Phe Ala Ala Pro Phe Ile
    1655                1660                1665

tct gcc gtt gca gca ttt gca tca tat gta gtt ggg ttc ttt aca 5049
Ser Ala Val Ala Ala Phe Ala Ser Tyr Val Val Gly Phe Phe Thr
    1670                1675                1680

ttt tct tta ttt tca tca tgt gta aca ata gct tct tca act tac 5094
Phe Ser Leu Phe Ser Ser Cys Val Thr Ile Ala Ser Ser Thr Tyr
    1685                1690                1695
 
tta tta tca aaa gtt gac aaa act ata aat aaa aat aag gag aga 5139
Leu Leu Ser Lys Val Asp Lys Thr Ile Asn Lys Asn Lys Glu Arg
    1700                1705                1710

ccg ttt tat tca ttt gta ttt gat atc ttt aag aat tta aaa cat 5184
Pro Phe Tyr Ser Phe Val Phe Asp Ile Phe Lys Asn Leu Lys His
    1715                1720                1725

tat tta caa caa atg aaa gaa aaa ttt agt aaa gaa aaa aat aat 5229
Tyr Leu Gln Gln Met Lys Glu Lys Phe Ser Lys Glu Lys Asn Asn
    1730                1735                1740

aat gta ata gaa gta aca aac aaa gct gag aaa aaa ggt aat gta 5274
Asn Val Ile Glu Val Thr Asn Lys Ala Glu Lys Lys Gly Asn Val
    1745                1750                1755

cag gta aca aat aaa acc gag aaa aca act aaa gtt gat aaa aat 5319
Gln Val Thr Asn Lys Thr Glu Lys Thr Thr Lys Val Asp Lys Asn
    1760                1765                1770

aat aaa gta ccg aaa aaa aga aga acg caa aaa tca aaa taa 5361
Asn Lys Val Pro Lys Lys Arg Arg Thr Gln Lys Ser Lys
    1775                1780                1785
<210> SEQ ID No 3
   <211> 1891
   <212> DNA
   <213> NUCLEOTIDE
<220>
   <221> CDS
   <222> (2)..(1891)
   <223>
<400> 3 
t aca tta act gaa agt gta gat gat aat aaa aat tta gaa gaa gcc gaa 49
Thr Leu Thr Glu Ser Val Asp Asp Asn Lys Asn Leu Glu Glu Ala Glu
1               5                   10                  15

gat ata aag gaa aat atc tta tta agt aat ata gaa gaa cca aaa gaa 97
Asp Ile Lys Glu Asn Ile Leu Leu Ser Asn Ile Glu Glu Pro Lys Glu
            20                  25                  30

aat att att gac aat tta tta aat aat att gga caa aat tca gaa aaa 145
Asn Ile Ile Asp Asn Leu Leu Asn Asn Ile Gly Gln Asn Ser Glu Lys
        35                  40                  45

caa gaa agt gta tca gaa aat gta caa gtc agt gat gaa ctt ttt aat 193
Gln Glu Ser Val Ser Glu Asn Val Gln Val Ser Asp Glu Leu Phe Asn
    50                  55                  60

gaa tta tta aat agt gta gat gtt aat gga gaa gta aaa gaa aat att 241
Glu Leu Leu Asn Ser Val Asp Val Asn Gly Glu Val Lys Glu Asn Ile
65                  70                  75                  80

ttg gag gaa agt caa gtt aat gac gat att ttt aat agt tta gta aaa 289
Leu Glu Glu Ser Gln Val Asn Asp Asp Ile Phe Asn Ser Leu Val Lys
                85                  90                  95

agt gtt caa caa gaa caa caa cac aat gtt gaa gaa aaa gtt gaa gaa 337
Ser Val Gln Gln Glu Gln Gln His Asn Val Glu Glu Lys Val Glu Glu
            100                 105                 110

agt gta gaa gaa aat gac gaa gaa agt gta gaa gaa aat gta gaa gaa 385
Ser Val Glu Glu Asn Asp Glu Glu Ser Val Glu Glu Asn Val Glu Glu
        115                 120                 125
 
aat gta gaa gaa aat gac gac gga agt gta gcc tca agt gtt gaa gaa 433
Asn Val Glu Glu Asn Asp Asp Gly Ser Val Ala Ser Ser Val Glu Glu
    130                 135                 140

agt ata gct tca agt gtt gat gaa agt ata gat tca agt att gaa gaa 481
Ser Ile Ala Ser Ser Val Asp Glu Ser Ile Asp Ser Ser Ile Glu Glu
145                 150                 155                 160

aat gta gct cca act gtt gaa gaa atc gta gct cca act gtt gaa gaa 529
Asn Val Ala Pro Thr Val Glu Glu Ile Val Ala Pro Thr Val Glu Glu
                165                 170                 175

att gta gct cca agt gtt gta gaa agt gtg gct cca agt gtt gaa gaa 577
Ile Val Ala Pro Ser Val Val Glu Ser Val Ala Pro Ser Val Glu Glu
            180                 185                 190

agt gta gct cca agt gtt gaa gaa agt gta gct gaa aat gtt gaa gaa 625
Ser Val Ala Pro Ser Val Glu Glu Ser Val Ala Glu Asn Val Glu Glu
        195                 200                 205

agt gta gct gaa aat gtt gaa gaa atc gta gct cca agt gtt gaa gaa 673
Ser Val Ala Glu Asn Val Glu Glu Ile Val Ala Pro Ser Val Glu Glu
    210                 215                 220

agt gta gct gaa aat gtt gaa gaa agt gta gct gaa aat gtt gaa gaa 721
Ser Val Ala Glu Asn Val Glu Glu Ser Val Ala Glu Asn Val Glu Glu
225                 230                 235                 240

agt gta gct gaa aat gtt gaa gaa agt gta gct gaa aat gtt gaa gaa 769
Ser Val Ala Glu Asn Val Glu Glu Ser Val Ala Glu Asn Val Glu Glu
                245                 250                 255

agt gta gct gaa aat gtt gaa gaa atc gta gct cca act gtt gaa gaa 817
Ser Val Ala Glu Asn Val Glu Glu Ile Val Ala Pro Thr Val Glu Glu
            260                 265                 270

agt gta gct cca act gtt gaa gaa att gta gct cca act gtt gaa gaa 865
Ser Val Ala Pro Thr Val Glu Glu Ile Val Ala Pro Thr Val Glu Glu
        275                 280                 285

agt gta gct cca act gtt gaa gaa att gta gtt cca agt gtt gaa gaa 913
Ser Val Ala Pro Thr Val Glu Glu Ile Val Val Pro Ser Val Glu Glu
    290                 295                 300

agt gta gct cca agt gtt gaa gaa agt gta gct gaa aat gtt gaa gaa 961
Ser Val Ala Pro Ser Val Glu Glu Ser Val Ala Glu Asn Val Glu Glu
305                 310                 315                 320

agt gta gct gaa aat gtt gaa gaa agt gta gct gaa aat gtt gaa gaa 1009
Ser Val Ala Glu Asn Val Glu Glu Ser Val Ala Glu Asn Val Glu Glu
                325                 330                 335

agt gta gct gaa aat gtt gaa gaa agt gta gct gaa aat gtt gaa gaa 1057
Ser Val Ala Glu Asn Val Glu Glu Ser Val Ala Glu Asn Val Glu Glu
            340                 345                 350

atc gta gct cca agt gtt gaa gaa atc gta gct cca act gtt gaa gaa 1105
Ile Val Ala Pro Ser Val Glu Glu Ile Val Ala Pro Thr Val Glu Glu
        355                 360                 365

agt gtt gct gaa aac gtt gca aca aat tta tca gac aat ctt tta agt 1153
Ser Val Ala Glu Asn Val Ala Thr Asn Leu Ser Asp Asn Leu Leu Ser
    370                 375                 380

aat tta tta ggt ggt atc gaa act gag gaa ata aag gac agt ata tta 1201
Asn Leu Leu Gly Gly Ile Glu Thr Glu Glu Ile Lys Asp Ser Ile Leu
385                 390                 395                 400
 
aat gag ata gaa gaa gta aaa gaa aat gta gtc acc aca ata cta gaa 1249
Asn Glu Ile Glu Glu Val Lys Glu Asn Val Val Thr Thr Ile Leu Glu
                405                 410                 415

aaa gta gaa gaa act aca gct gaa agt gta act act ttt agt aat ata 1297
Lys Val Glu Glu Thr Thr Ala Glu Ser Val Thr Thr Phe Ser Asn Ile
            420                 425                 430

tta gag gag ata caa gaa aat act att act aat gat act ata gag gaa 1345
Leu Glu Glu Ile Gln Glu Asn Thr Ile Thr Asn Asp Thr Ile Glu Glu
        435                 440                 445

aaa tta gaa gaa ctc cac gaa aat gta tta agt gcc gct tta gaa aat 1393
Lys Leu Glu Glu Leu His Glu Asn Val Leu Ser Ala Ala Leu Glu Asn
    450                 455                 460

acc caa agt gaa gag gaa aag aaa gaa gta ata gat gta att gaa gaa 1441
Thr Gln Ser Glu Glu Glu Lys Lys Glu Val Ile Asp Val Ile Glu Glu
465                 470                 475                 480

gta aaa gaa gag gtc gct acc act tta ata gaa act gtg gaa cag gca 1489
Val Lys Glu Glu Val Ala Thr Thr Leu Ile Glu Thr Val Glu Gln Ala
                485                 490                 495

gaa gaa gag agc gaa agt aca att acg gaa ata ttt gaa aat tta gaa 1537
Glu Glu Glu Ser Glu Ser Thr Ile Thr Glu Ile Phe Glu Asn Leu Glu
            500                 505                 510

gaa aat gca gta gaa agt aat gaa aaa gtt gca gag aat tta gag aaa 1585
Glu Asn Ala Val Glu Ser Asn Glu Lys Val Ala Glu Asn Leu Glu Lys
        515                 520                 525

tta aac gaa act gta ttt aat act gta tta gat aaa gta gag gaa aca 1633
Leu Asn Glu Thr Val Phe Asn Thr Val Leu Asp Lys Val Glu Glu Thr
    530                 535                 540

gta gaa att agc gga gaa agt tta gaa aac aat gaa atg gat aaa gca 1681
Val Glu Ile Ser Gly Glu Ser Leu Glu Asn Asn Glu Met Asp Lys Ala
545                 550                 555                 560

ttt ttt agt gaa ata ttt gat aat gta aaa gga ata caa gaa aat tta 1729
Phe Phe Ser Glu Ile Phe Asp Asn Val Lys Gly Ile Gln Glu Asn Leu
                565                 570                 575

tta aca ggt atg ttt cga agt ata gaa acc agt ata gta atc caa tca 1777
Leu Thr Gly Met Phe Arg Ser Ile Glu Thr Ser Ile Val Ile Gln Ser
            580                 585                 590

gaa gaa aag gtt gat ttg aat gaa aat gtg gtt agt tcg att tta gat 1825
Glu Glu Lys Val Asp Leu Asn Glu Asn Val Val Ser Ser Ile Leu Asp
        595                 600                 605

aat ata gaa aat atg aaa gaa ggt tta tta aat aaa tta gaa aat att 1873
Asn Ile Glu Asn Met Lys Glu Gly Leu Leu Asn Lys Leu Glu Asn Ile
    610                 615                 620

tca agt act gaa ggc gaa 1891
Ser Ser Thr Glu Gly Glu
625                 630
<210> SEQ ID No 4
   <211> 28
   <212> DNA
   <213> NUCLEOTIDE
<400> 4
   gtgatgaact ttttaatgaa ttattaaa 28
<210> SEQ ID No 5
   <211> 29
   <212> DNA
   <213> NUCLEOTIDE
<400> 5
   tgttgttctt gttgaacact ttttactaa 29
<210> SEQ ID No 6
   <211> 25
   <212> DNA
   <213> NUCLEOTIDE
<400> 6
   ggtatcgaaa ctgaggaaat aaagg 25
<210> SEQ ID No 7
   <211> 24
   <212> DNA
   <213> NUCLEOTIDE
<400> 7
   catagcagga acatcaacat ccac 24

## Description

Les parasites responsables du paludisme chez l'homme présentent chez l'hôte humain des morphologies différentes et expriment des antigènes différents en fonction de leur localisation dans l'organisme. Les différences morphologiques et antigéniques de ces parasites au cours de leurs cycles de vie chez l'homme, permettent de définir différents stades de développement dans le foie et dans le sang : le sporozoïte, forme infectieuse injectée par le moustique vecteur, se transforme rapidement en schizonte dans les hépatocytes de l'hôte pour infecter ensuite les erythrocytes. La localisation intra-hépatique de P.falciparum se traduit par l'expression d'un groupe d'antigènes spécifiques de ce stade de développement et très immunogènes dans les condition naturelles d'exposition à la maladie. Cette phase, cliniquement silencieuse, est actuellement la seule contre laquelle on peut induire expérimentalement chez l'homme une immunité très forte, stérilisante, par injection de sporozoïtes irradiés, capables de pénétrer dans l'hépatocyte et de s'y développer, mais sans pouvoir aboutir au stade sanguin de la maladie. C'est pourquoi les inventeurs ont focalisé l'essentiel de leurs efforts sur ces deux stades pré-érythrocytaires. Mais ces stades sont également les plus délicats à étudier, et donc les moins connus, puisque l'obtention de matériel biologique est difficile, voire impossible, que le seul modèle d'étude in vitro possède un rendement très faible et que le meilleur modèle animal reste le chimpanzé, d'utilisation limitée et onéreuse.

Afin d'accéder aux antigènes des stades pré-érythrocytaires, les inventeurs ont utilisé des sérums d'individus résidant en zone d'endémie depuis 25 ans mais sous prophylaxie permanente à la chlroroquine. Ces individus étaient régulièrement soumis aux piqûres de moustiques infectés mais ne développaient aucune infection sanguine complète. Leur sérum contenait donc des anticorps essentiellement dirigés contre les stades pré-érythrocytaires, ce qui fut vérifié par Immuno-Fluorescence (IF) et Western Blot sur les 3 stades du parasite.

L'utilisation de ces sérums pour le criblage d'une banque d'ADN génomique du clone parasitaire de P.falciparum, construite en vecteurs d'expression dans un phage lambda gt11 (V. Rosario, Science 212, 1981, p.1037-1038 ; et Thaithong et al, Transactions of Royal Society of Tropical Medicine and Hygien, 1984, 78 : 242-245) a conduit à la mise en évidence de polypeptides du stade pré-erythrocytaire, notamment les polypeptides SALSA (pour Sporozoïte Liver Stage Antigen) décrits dans EP A-0407230 et le LSA 1 (pour Liver Stage Antigen) décrit dans W0 92/13884. La présente invention est relative à de nouvelles molécules polypeptidiques spécifiques du stade pré-érythrocytaire et à leur utilisation à titre de principe actif de vaccin anti-palustre ou dans des méthodes de diagnostics de la maladie.

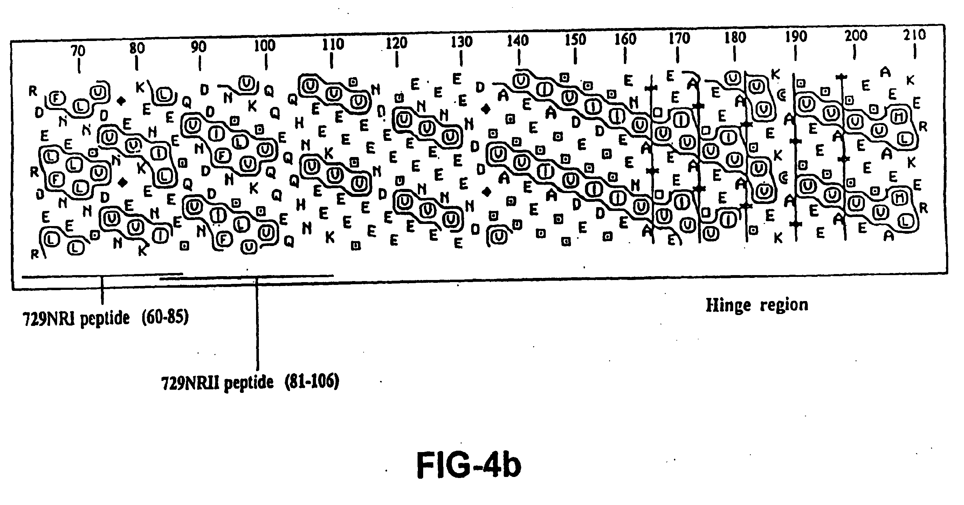

L'invention résulte de la mise en évidence par les inventeurs des propriétés particulières d'un antigène particulier appelé LSA-3 et de ses fragments, qui apparaissent comme des candidats à fort potentiel pour réaliser un vaccin anti-palustre et ceci pour les raisons suivantes :
a) lorsque une fraction de LSA-3 était utilisée en combinaison avec un autre antigène du même stade de développement du parasite, comme le LSA-1, pour immuniser des chimpanzés, l'animal répondant aux deux molécules ou uniquement à LSA-3 présente la caractéristique de ne pas avoir de parasites dans le sang, d'avoir une diminution importante des parasites dans le foie, et de manifester un recrutement de cellules mononucléées important indiquant une réponse en immunité cellulaire ;
b) en zone d'endémie, on observe une très nette corrélation entre la protection des individus contre les infections naturelles par sporozoïtes et leurs réponses en anticorps contre LSA-3 ;
c) chez huit volontaires humains immunisés par injection de sporozoïtes irradiés, des anticorps dirigés contre LSA-3 sont trouvés chez chacun des quatre individus résistant à une infection par sporozoïtes et chez aucun des quatre autres volontaires ayant développé un infection sanguine ;
d) des anticorps obtenus contre le peptide DG729 dans WO 92/13884 déjà décrit donnent une réaction croisée avec les stades sporozoïtes et hépatiques du parasite murin P yoelii ce qui permet une exploitation significative du modèle souris. In vitro les anticorps humains immuno purifiés sur DG729 sont capables, même à très faibles concentrations, de bloquer la pénétration des sporozoïtes de P yoelii dans les hépatocytes murins. In vivo les souris immunisées par DG729 sont totalement ou partiellement protégées contre les infections par les sporozoïtes de P.yoelii;
e) enfin, certains épitopes notamment dans les parties non répétées de la molécule stimulent la sécrétion d'interféron γ par les monocytes, ce médiateur permettant d'inhiber le développement intra-hépatique du parasite (S. Mellouk et al, The Jour. of Imun. 139, 4192-4195. 1987).
f) la séquence de la région de LSA-3 correspondant à un (lipo) peptide NRII a été analysée dans 27 échantillons : 4 souches de laboratoire (NF54, K1, Palo Alto, T9/96), 3 isolats malgaches, 3 isolats birmans, 5 isolats brésiliens, 7 isolats ivoiriens et 5 isolats thaïs. Aucune mutation n'a été observée sur les 300 paires de bases analysées, soit 100 % de conservation dans cette région immunologiquement importante contenant un ou plusieurs épitopes B, Th et CTL.
g) des informations sur la structure de l'antigène, et notamment d'un peptide RE, et plus particulièrement sur la région répétée centrale à partir de laquelle le peptide RE a été conçu et qui comporte un ou plusieurs épitopes B majeurs, ont été obtenues à partir du Hydrophobic Cluster Plot de la séquence disponible dans le clone T9/96 (630 acides aminés), (Gaboriot et al., (1987) : Hydrophobic cluster analysis : an efficient new way to compare and analyse amino acide séquences, FEBS Letters, 224 : 149-155) ; cette méthode prédit une très forte propension à l'organisation en hélice-α. La région répétée présente une extraordinaire régularité dans l'espacement des résidus de Valine et d'Isoleucine, alternant avec des résidus acides ou de Proline. La disposition des groupements hydrophobes à la surface de cette hélice évoque une bordure hydrophobe se décalant graduellement d'une face de l'hélice à l'autre, suivant une orientation générale constante le long de la molécule, et probablement en relation avec une structure ou un empaquetage « coiled-coil » comme cela apparaît dans la figure 4b qui représente le HCP (Hydrophobic Cluster Plot) de la séquence peptidique du clone DG729.
h) après avoir démontré qu'il existait une très large gamme de réponses immunitaires à l'antigène LSA-3, nous avons analysé la capacité des cellules répondeuses à se localiser autour des parasites dans le foie. Chez les souris immunisées par les antigènes recombinants, l'injection par voie intraportale de chacun des peptides absorbés sur des billes de polystyrène de 10µm permet de visualiser au bout de 48 heures un afflux de lymphocytes autour de l'antigène (mimant le parasite), puis au 5ème jour, un important recrutement de cellules appartenant à la lignée macrophagique.

Toutes ces propriétés dont certaines seront démontrées en détail dans les expériences décrites plus loin montrent que l'antigène LSA-3 présente à la fois une bonne antigénicité et une bonne immunogénicité.

Les inventeurs ont pu confirmer et préciser la spécificité des stades d'expression de la molécule ; au niveau des sporozoïtes, cette expression a été confirmée par l'immunofluorescence en surface de plusieurs souches et isolats. En analyse par « Western Blot » (ou lmmunoempreinte), la molécule LSA-3 apparaît comme une protéine d'un poids moléculaire de 200 000 daltons. Si les ARN messagers de sporozoïtes n'ont pu être obtenus en quantité suffisante pour une analyse en « northern blot », des expériences de PCR inverse ont confirmé l'expression de LSA-3 à ce stade. Au niveau des hépatocytes infectés, LSA-3 est observé dans la vacuole parasitophore du parasite par immunofluorescence à l'aide d'anticorps contre les régions répétées et non répétées de la protéine, ainsi qu'en microscopie électronique.

Un fragment de LSA-3 dénommé 729S ainsi que trois peptides dénommés NRI et NRII inclus dans la partie non répétée, et 729 R inclus dans la partie répétée ont été décrits dans la demande WO 92/13884. Néanmoins ce document n'évoque pas les propriétés particulières mentionnées ci-dessus ni d'autres fragments de LSA-3 qui pourraient être soit plus longs soit plus courts, inclus ou combinés avec ces fragments qui présenteraient des propriétés particulièrement intéressantes pour une utilisation dans des vaccins.

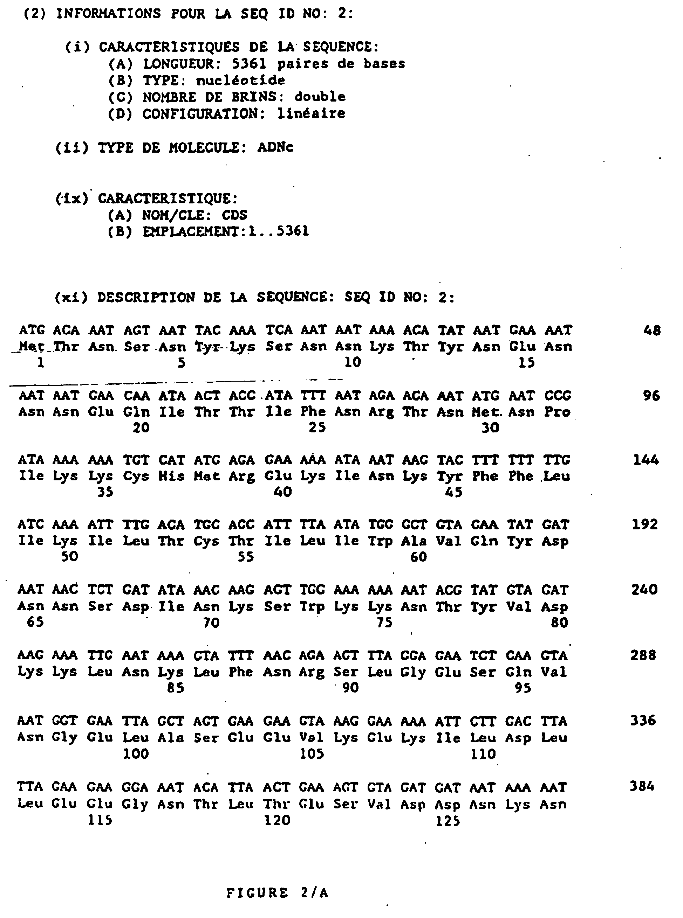

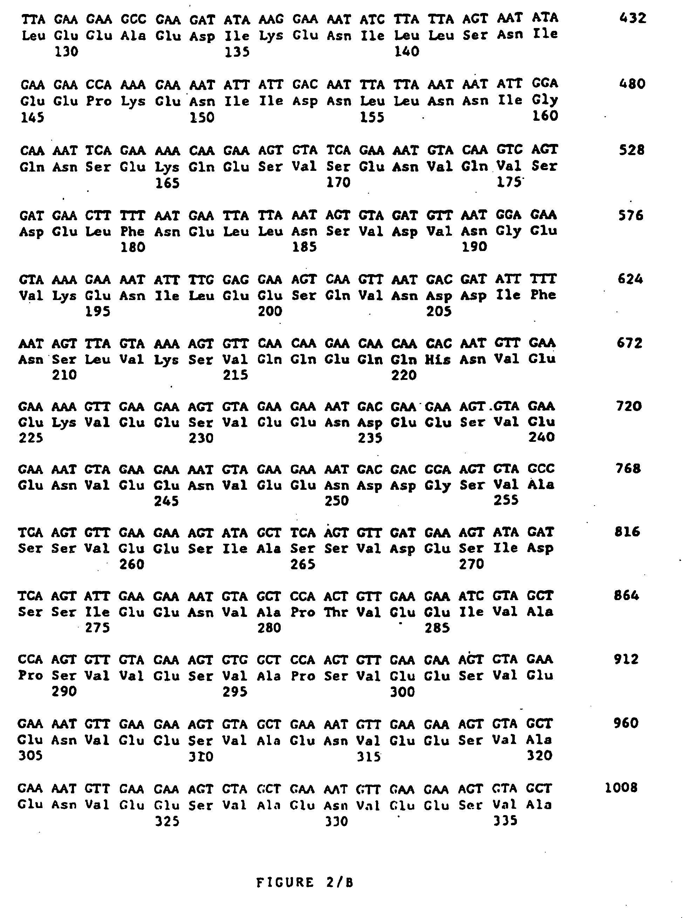

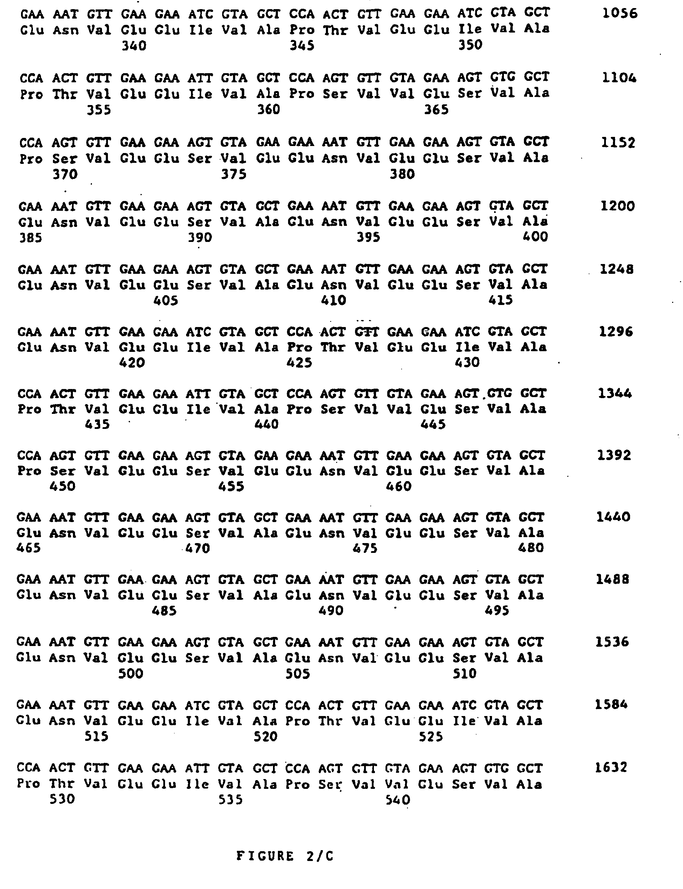

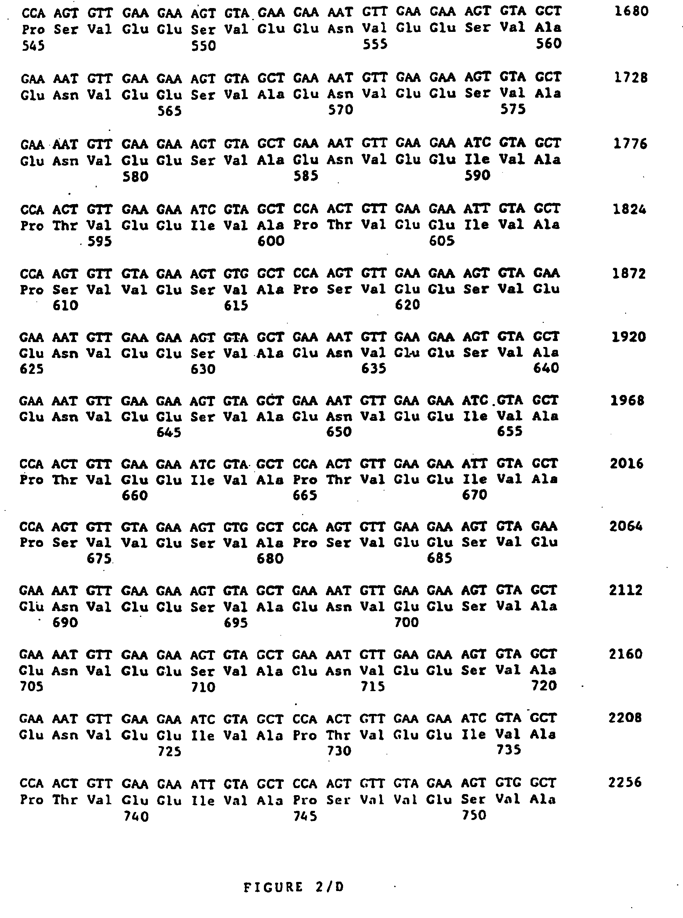

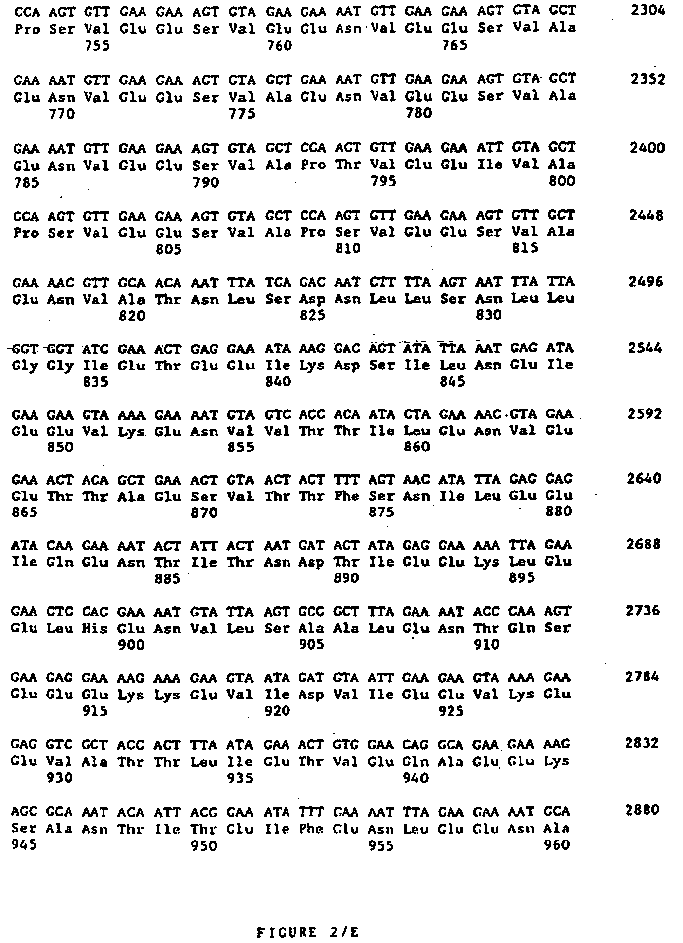

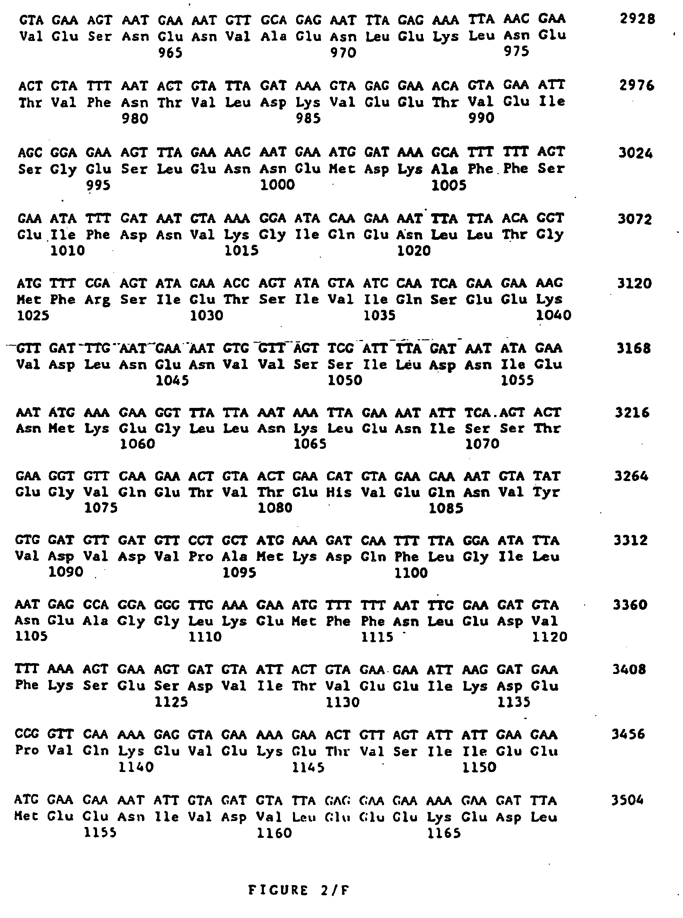

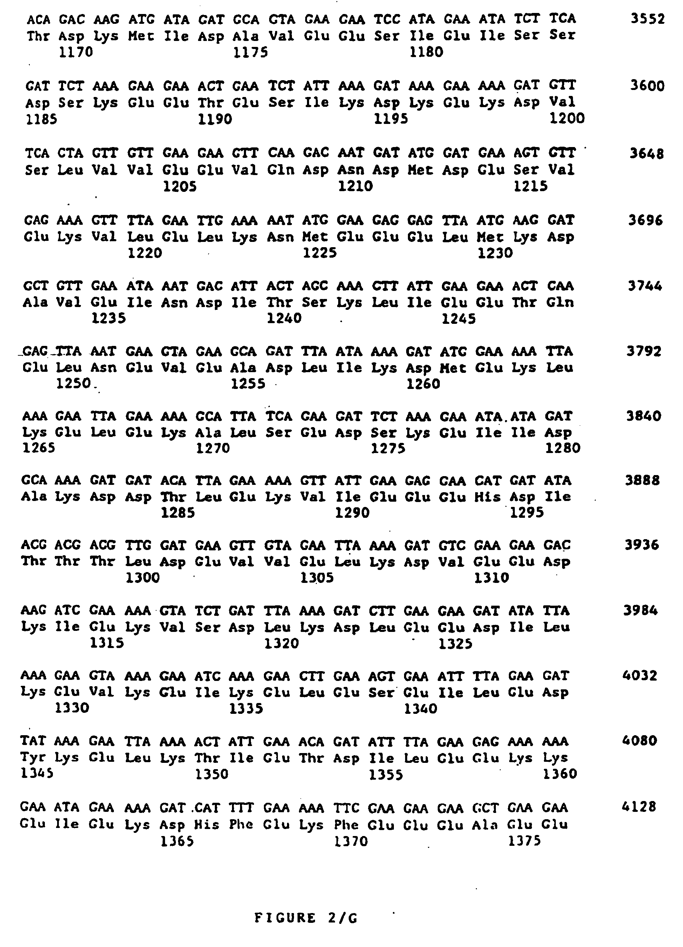

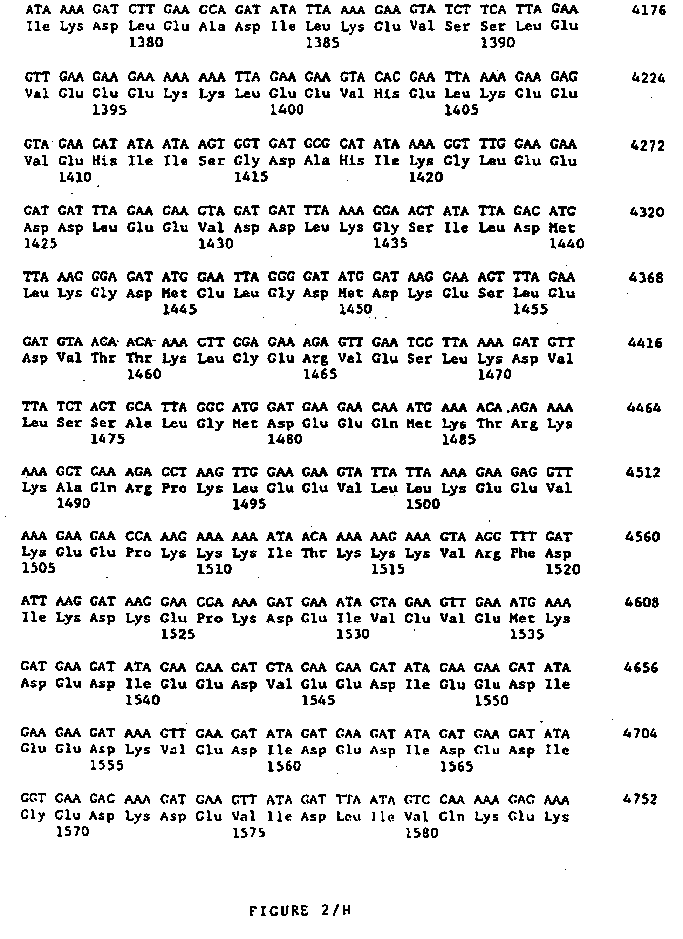

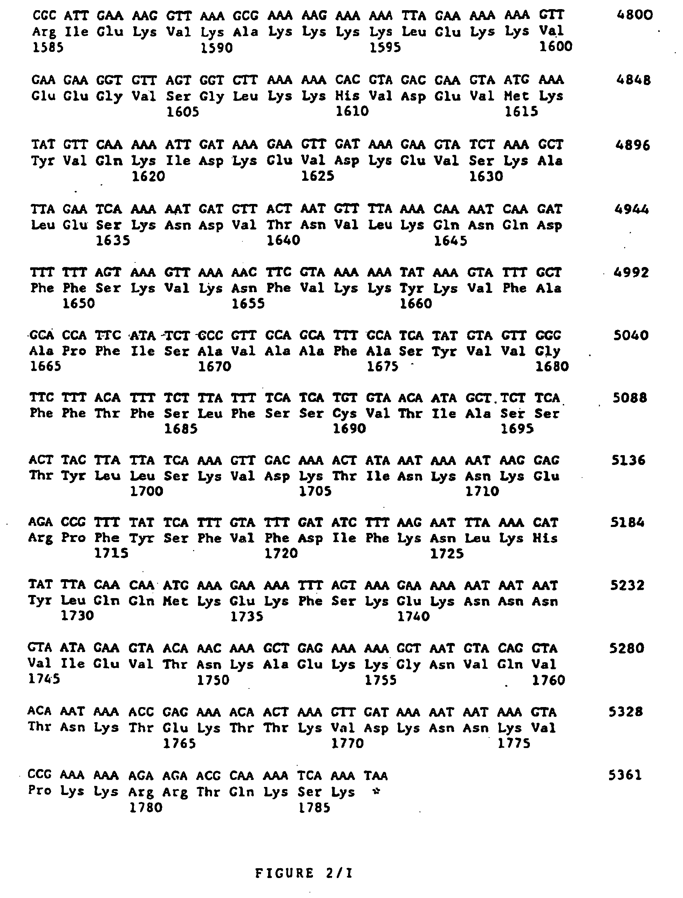

La présente demande décrit des molécules polypeptidiques contenant au moins dix acides aminés consécutifs de la séquence d'acides aminés montrée dans la figure 2 et dénommée SEQ ID n° 2, et représentant LSA-3, les polypeptides suivants étant exclus :

-RDELFNELLNSVDVNGEVKENILEESQVNDDIFNSLVKSVQQEQQHNVEE
VEESVEENDEESVEENVEENVENNDDGSVASSVEESIASSVDESIDSSIE -
ENVAPTVEEIVAPTVEEIVAPSVVEKCAPSVEESVAPSVEESVAEMLKER
(729S)

- RDELFNELLNSVDVNGEVKENILEESQVNDDIFNSLVKSVQQEQQHN
- DELFNELLNSVDVNGEVKENILEESQ, (NR I)
- LEESQVNDDIFSNSLVKSVQQEQQHNV, (NR II)
- VESVAPSVEESVAPSVEESVAENVEESV. (729 RE)

D'autres molécules d'intérêt contiennent au moins 20 acides aminés consécutifs ou au moins 50.

L'ensemble de ces polypeptides et la molécule LSA-3 sont dans tout ce qui suit « polypeptides d'intérêt selon la demande ».

L'invention concerne un conjugué où un résidu lipidique en C16 ou C18 est couplé par un pont lysine aux peptides NRI, NRII, 729RE ou CT1. Elle concerne également un conjugué immunogene dont la séquence polypeptidique est d'origine d'un isolat de *P.falciparum* et présente au moins 70% d'homologie avec celle d'un conjugué.

Les résultats expérimentaux et les comparaisons de séquence non répétées entre différents isolats de P.falciparum indiquent l'existence d'une homologie d'au moins 70% entre des antigènes équivalents du stade hépatique du parasite. Aussi toute molécule peptidique présentant au moins 70% d'homologie avec une quelconque des molécules définies cidessus fait partie de la présente description ainsi que celles présentant au 70% d'homologie avec la séquence suivante :
Leu Leu Ser Asn Ile Glu Glu Pro Lys Glu Asn Ile Ile Asp Asn Leu Leu Asn Asn Ile (CT1)
comprise entre les acide aminés 140-159 de K1 ou 23-42 du T9/96.

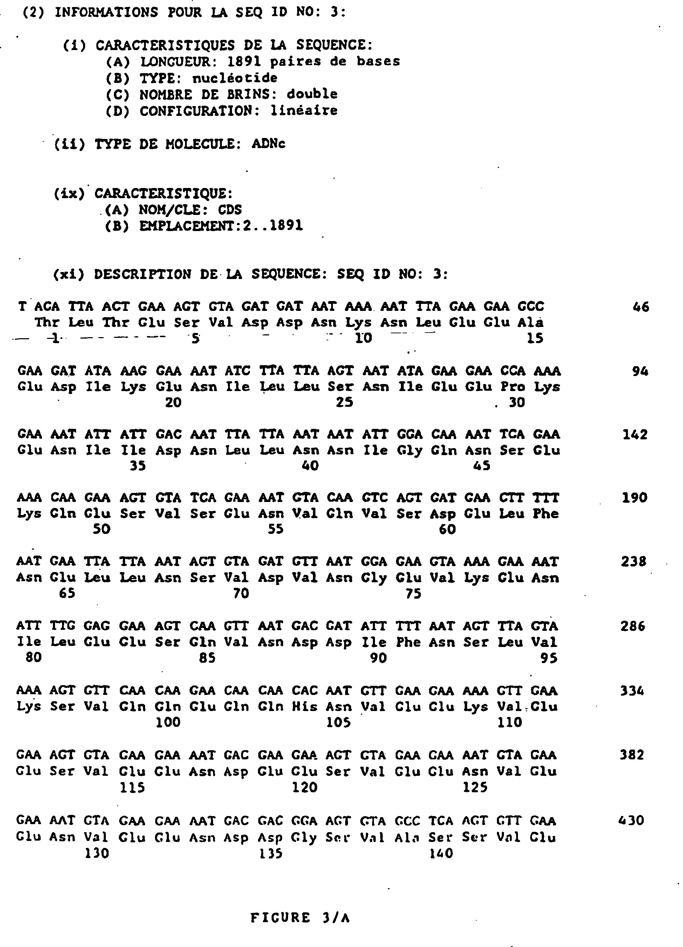

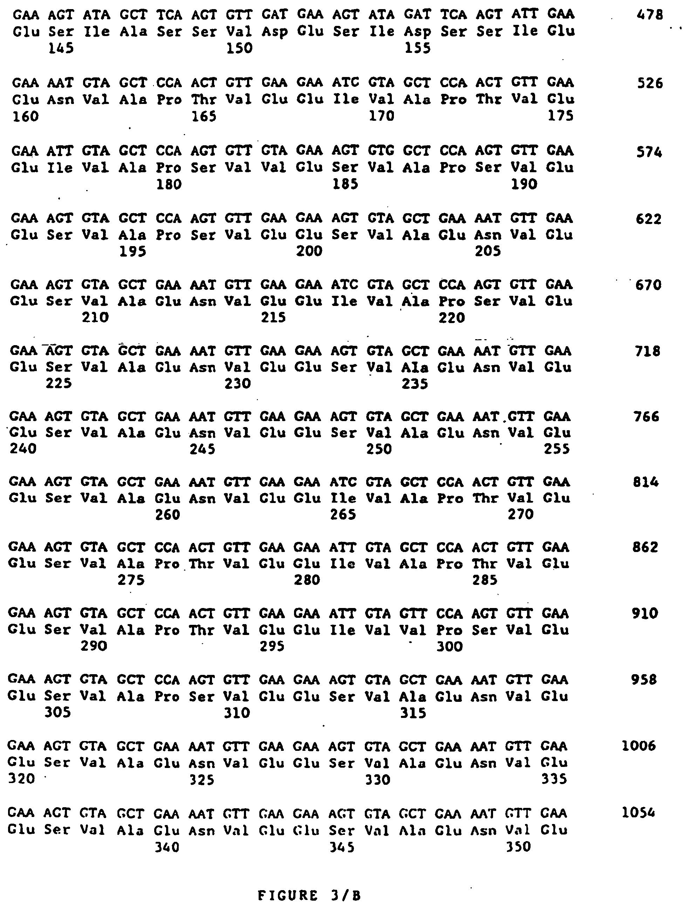

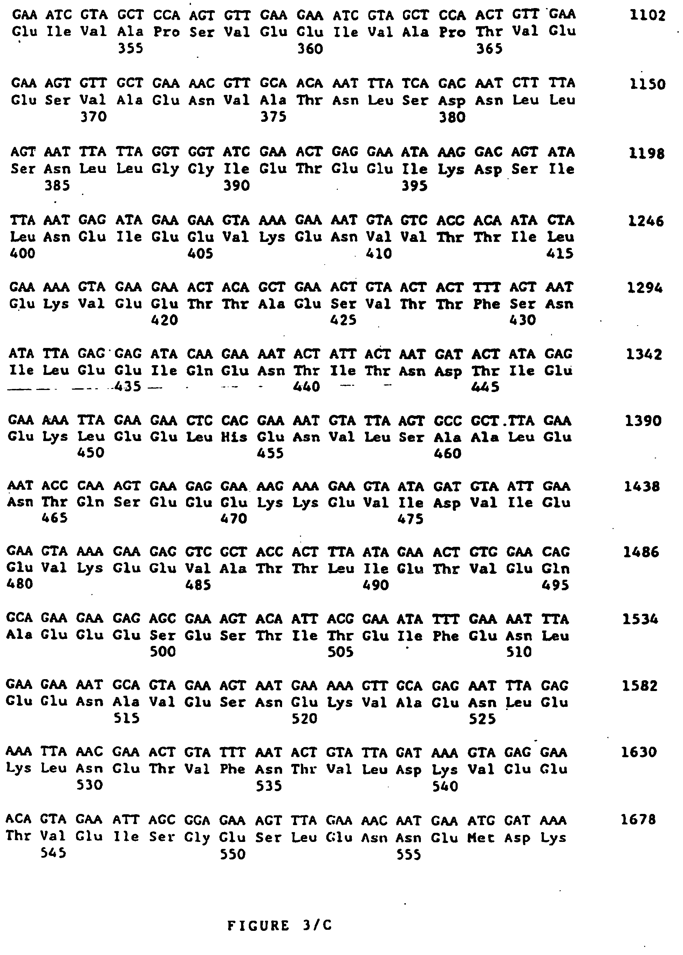

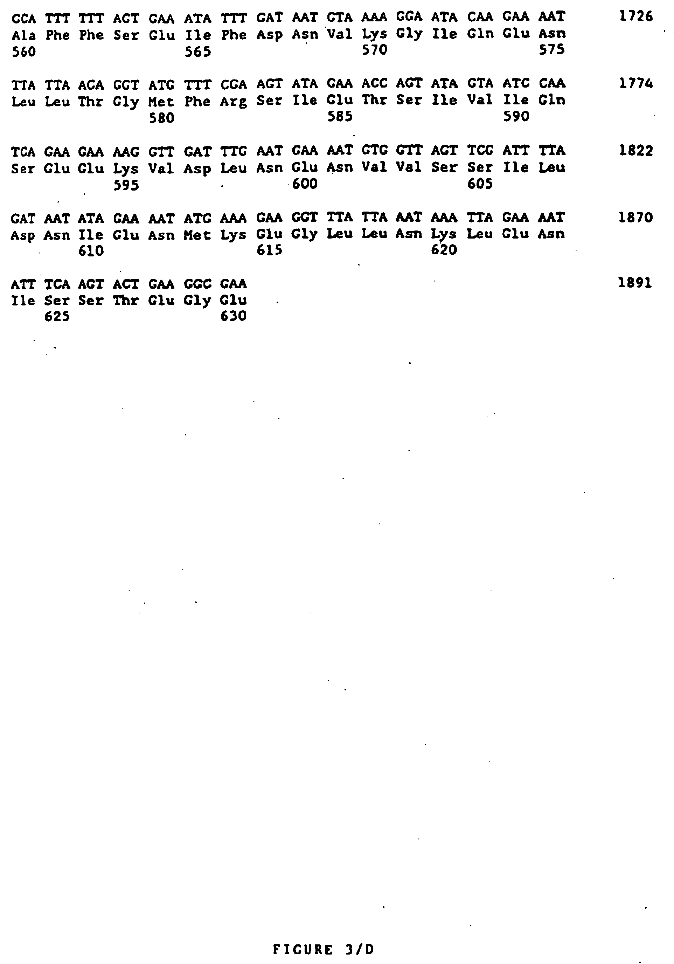

De la même façon sont décrites ici les molécules polypeptidiques présentant au moins 70% d'homologie avec la séquence représentée dans la figure 3 qui représente une partie de LSA-3 dans T9/96 : L'ADN de cet isolat de P.falciparum a été digéré par des enzymes de restriction puis cloné dans lambda gt11 et a ainsi permis de constituer la génothèque de cette isolat déjà décrite plus haut.

Des conjugués constitués d'un polypeptide issu de LSA-3 lié de façon covalente par un pont lysine à des résidus lipidiques saturés ou insaturés font également partie de l'invention plus particulièrement lorsque le résidu lipidique est un palmitoyl ou un palmityl ou un oléyl. Des résidus en C16 ou C18 ont ainsi été couplés par un pont lysine aux peptides NRI. NRII, 729 RE et CT1 déjà représentés ci-dessus. La méthode de synthèse utilisée pour ces conjugués est décrite dans Bourgault, Journal of Immunology, 149, 3416 (1992) et Rouaix, Vaccine, 12, 1209 (1994).

L'invention porte également sur des compositions immunogènes contenant au moins un des conjugués décrits ci-dessus, ainsi que sur les vaccins contenant ces compositions immunogènes. D'autres épitopes immunogènes notamment LSA-1, SALSA, STARP ont déjà été décrits dans EP A-0407230 et dans WO 92/13884 Les compositions de vaccin selon l'invention peuvent contenir de façon avantageuse un mélange de peptides immunogènes issus de LSA-3 et des peptides ou antigènes issus de LSA-1, SALSA ou STARP ; un mélange plus particulièrement intéressant pourrait être celui constitué d'une part de NRI, NRII ou de LSA-3 entier, couplés ou non à un résidu lipidique, et d'autre part des peptides SALSA-1 ou SALSA2 ou l'antigène SALSA couplé ou non à un résidu lipidique.

Toutes les molécules polypeptidiques répondant à la définition ci-dessus et présentant au moins 70% d'homologie avec les polypeptides LSA-3, CT1, NRI, NRII ou 729RE, peuvent être combinées de façon homologue ou hétérologue avec d'autres séquences peptidiques ou issues d'un autre antigène des différents stades de P.falciparum.

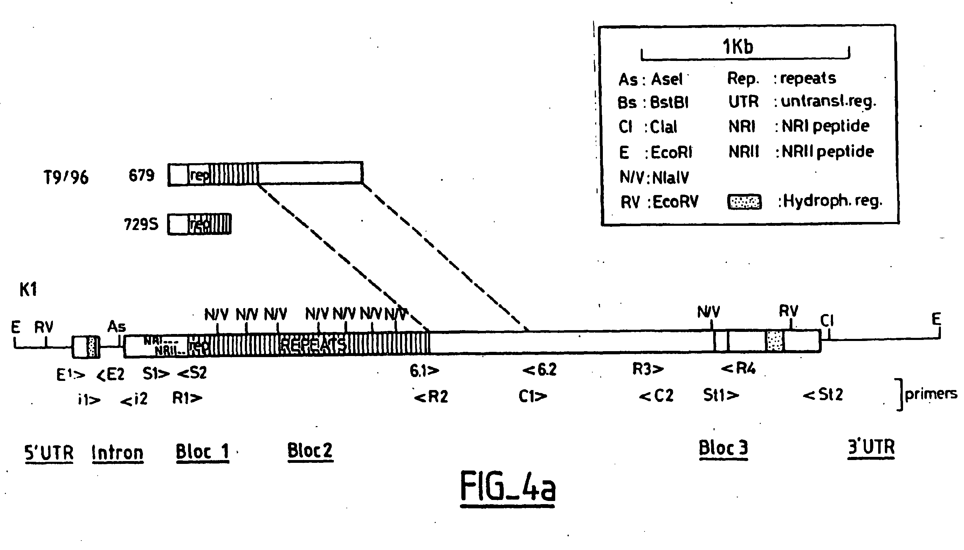

Par 70 % d'homologie des séquences, il est bien entendu qu'il s'agit d'une homologie de séquences par rapport à l'un quelconque des isolats dont la séquence est connue ou susceptible d'être connue, et non pas globalement entre l'ensemble des isolats. En effet, la région répétée centrale de LSA-3 (bloc 2 de la figure 4) présente un nombre variable de séquences répétées responsables d'une variabilité d'un isolat à l'autre comme l'indique d'ailleurs la représentation de la figure 4 dans laquelle la différence de longueur entre les parties répétées du bloc 2 des isolats T9/96 et K1 est flagrante bien que les tétrapeptides qui constituent cette région répétée (VEES, VEEN, VEEI, VAPS, VAPT, etc...) soient très bien conservés. En revanche, les séquences répétées du bloc 1 sont parfaitement conservées entre les deux isolats. Aussi compte tenu de la variabilité intrinsèque de ce bloc 2 d'un isolat à l'autre, la définition 70 % d'homologie s'entend pour l'antigène LSA-3 des différents isolats à l'exclusion des séquences répétées du bloc 2.

Sont décrits également les anticorps polyclonaux ou monoclonaux qui reconnaissent spécifiquement les molécules polypeptidiques de l'invention.

Ces molécules d'intérêt peuvent être utilisées pour la mise en oeuvre de méthodes de diagnostic et la fabrication de trousses permettant de détecter l'existence de l'infection par P.falciparum : cette méthode peut être soit un dosage d'anticorps spécifiques circulant par la mise en oeuvre de méthodes sérologiques classiques par mise en contact d'un des antigènes ci-dessus avec un fluide biologique de l'individu en question, soit des méthodes de dosages d'antigènes en utilisant des anticorps polyclonaux ou monoclonaux obtenus par des méthodes classiques d'obtention de tels anticorps par les antigènes correspondants. Dans les coffrets ou kits des diagnostics de l'invention, les réactifs permettant la détection des complexes antigènes/anticorps produits, pouvant également porter un marqueur ou être susceptibles d'être reconnus à leur tour par un réactif marqué sont présents. Selon que l'on souhaite réaliser un test antigène ou un test sérologique, le kit comprend soit les anticorps soit les antigènes de l'invention.

La présente demande décrit également toutes les séquences de nucléotides codant pour un polypeptide de l'invention ainsi que sur tout acide nucléique recombinant contenant au moins une séquence de nucléotides de l'invention, insérée dans un acide nucléique hétérologue vis à vis de ladite séquence de nucléotides.

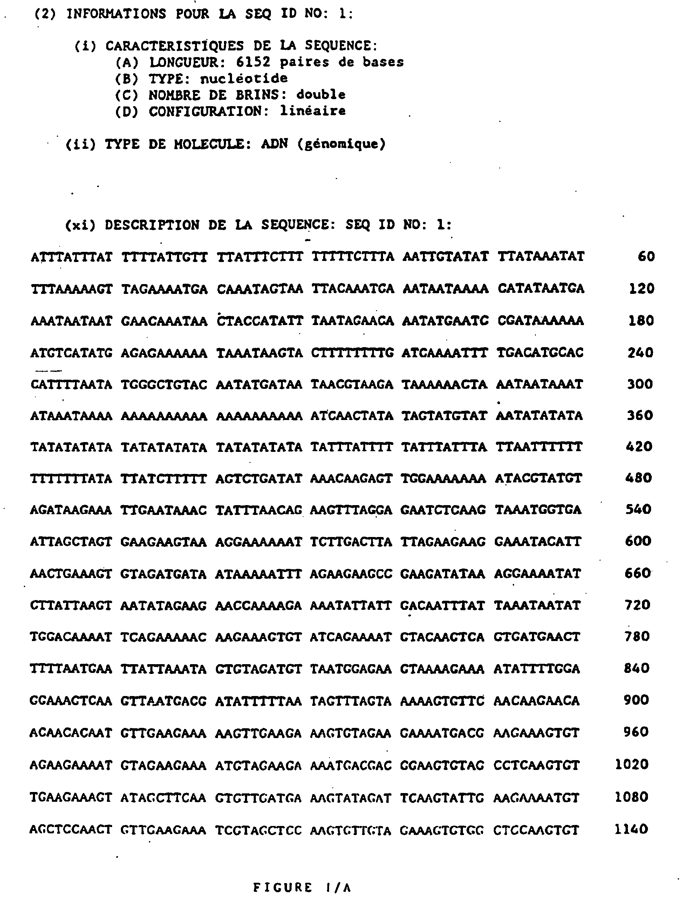

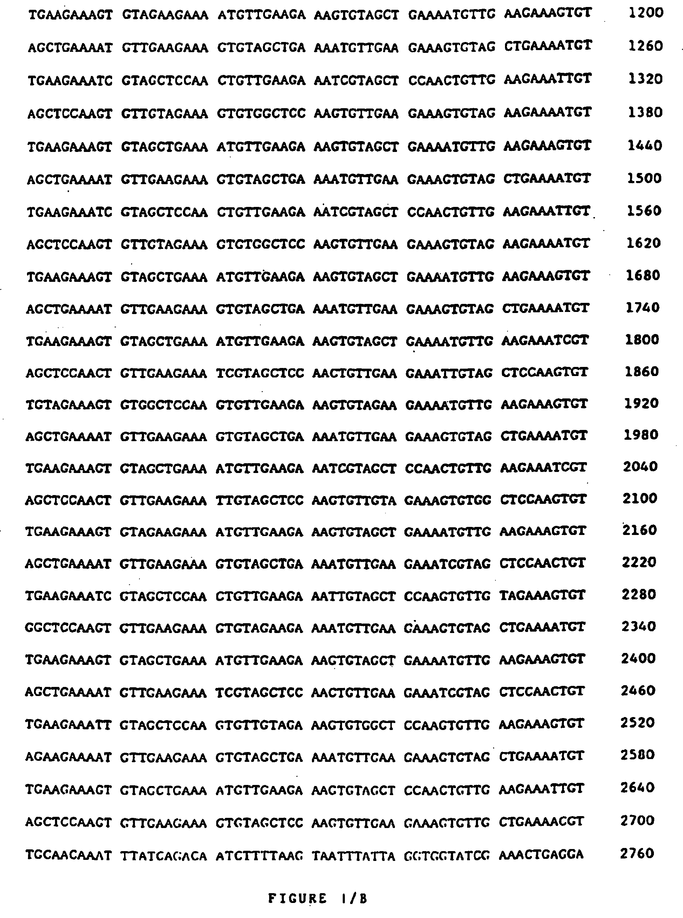

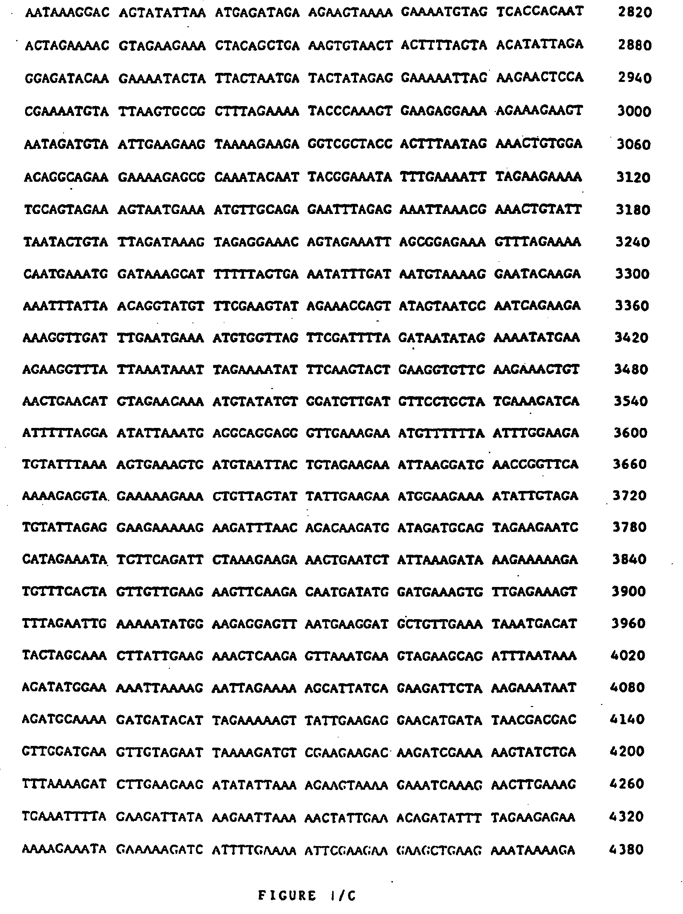

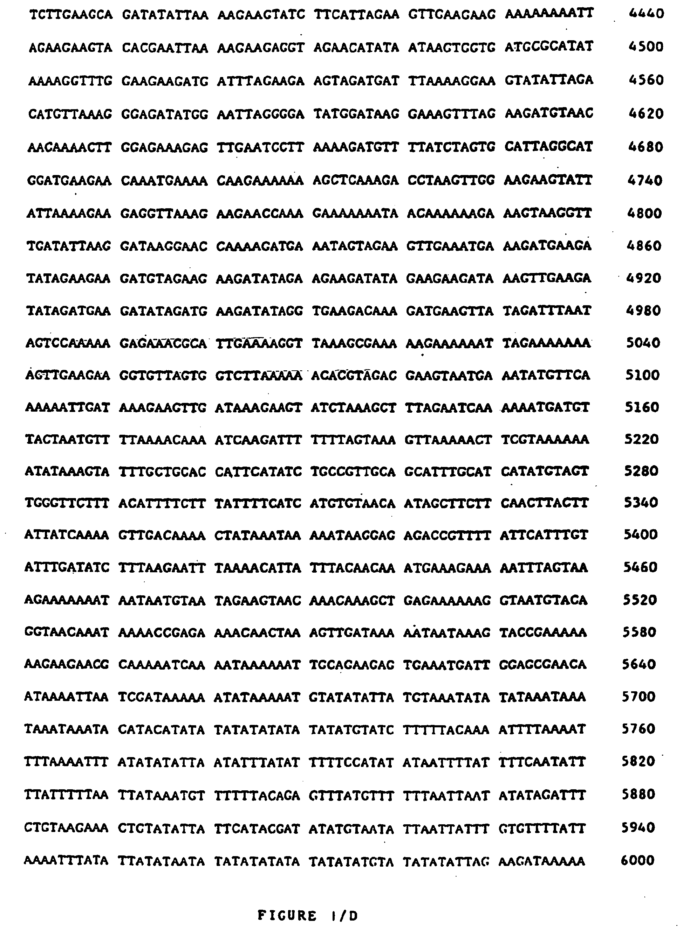

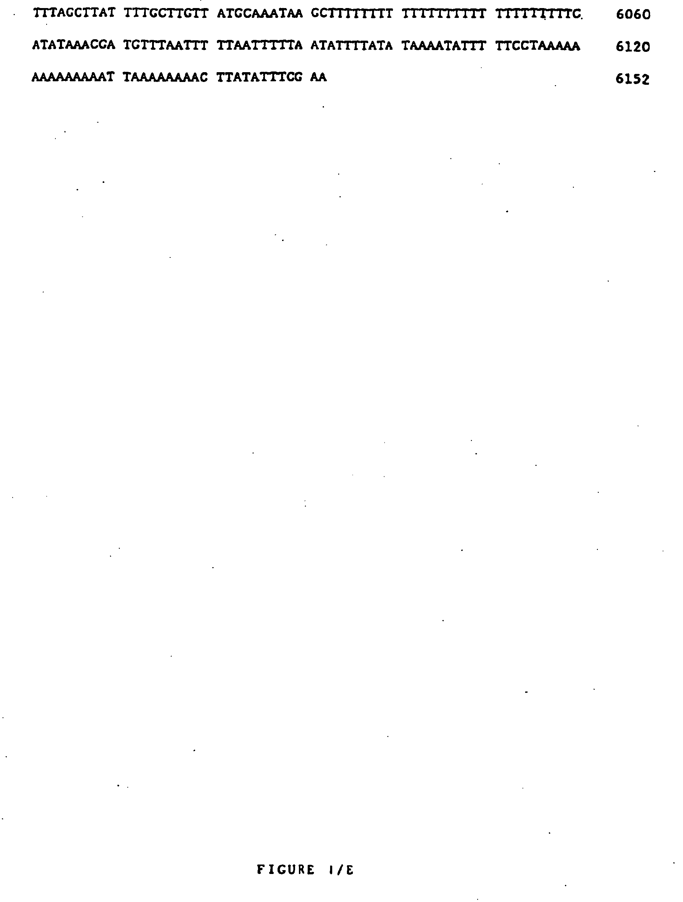

Son décrites aussi ici les séquences d'acides nucléiques codant pour LSA-3 ou ses fragments immunogènes et répondant à l'une des définitions suivantes :
(a) l'enchaînement de nucléotides tel que représenté dans SEQ ID n° 1 de la figure 1. ou
(b) l'enchaînement de nucléotides représenté dans SEQ ID n° 2 de la figure 2,
(c) un enchaînement présentant au moins 70% d'homologie avec celui de la figure 1, ou de la figure 2 ou,
(d) un enchaînement de nucléotides complémentaires à ceux présentés en (a), (b) ou (c).

Par codant pour LSA-3, on entend tant le gène représenté dans SEQ ID n° 1 de la figure 1 que le cDNA représenté dans SEQ ID n° 2 de la figure 2.

Est décrit plus particulièrement un acide nucléique recombinant, dans lequel la séquence de nucléotides d'intérêt est précédée d'un promoteur (notamment un promoteur inductible) sous le contrôle duquel la transcription de ladite séquence est susceptible d'être effectuée et, le cas échéant, suivie d'une séquence codant pour des signaux de terminaison de la transcription.

Est décrite également la séquence codante issue du clone T9/96, représenté sur la figure 3 par la SEQ ID n° 3.

Dans cette séquence, le fragment CT1 est compris entre le nucléotide 67. et 126, et le fragment 679 commerce au nucléotide 206 et le fragment 729 RE est compris entre les nucléotides 547 et 630.

Est décrit enfin tout vecteur recombinant, utilisé en particulier pour le clonage d'une séquence nucléotidique de l'invention, et/ou pour l'expression du polypeptide codé par cette séquence, et caractérisé en ce qu'il contient un acide nucléique recombinant, tel que défini ci-dessus, en l'un de ses sites non essentiel pour sa réplication.

A titre d'exemple de vecteur sus-mentionné, on citera les plasmides, les cosmides, les phages ou les virus.

Est décrit plus particulièrement le plasmide pK 1.2. déposé à la CNCM sous le n°-1573.

Est décrit également un procédé de préparation d'un polypeptide décrit dans la demande, par transformation d'un hôte cellulaire à l'aide d'un vecteur recombinant de type sus-indiqué, suivie de la mise en culture de l'hôte cellulaire ainsi transformé, et de la récupération du polypeptide dans le milieu de culture.

Ainsi, est décrit tout hôte cellulaire transformé par un vecteur recombinant tel que défini ci-dessus, et comprenant les éléments de régulation permettant l'expression de la séquence de nucléotides codant pour un polypeptide décrit dans la demande.

La demande décrit de même sur des amorces d'ADN (ou d'ARN) utilisables dans le cadre de la synthèse de séquences nucléotidiques et/ou polypeptidiques d'intérêt, par la technique du PCR (Polymerase Chain Reaction) ou toute autre méthode aujourd'hui connue pour amplifier les acides nucléiques telles la LCR, CPR, ERA, SPA, NASBA, etc...

La demande décrit une amorce d'ADN ou d'ARN, caractérisée en ce qu'elle est constituée d'environ 10 à 25 nucléotides, identiques ou complémentaires aux 10 à 25 premiers nucléotides de la séquence de nucléotides codant pour une séquence peptidique d'intérêt selon la demande ou identiques aux 10 à 25 deniers nucléotides de ladite séquence.

Ainsi la présente demande décrit également un procédé de préparation d'un polypeptide d'intérêt selon la demande comprenant les étapes suivantes :
- le cas échéant, l'amplification préalable par des techniques classiques de la quantité de séquences de nucléotides codant pour ledit polypeptide à l'aide de deux amorces d'ADN choisies de manière appropriée,
- la mise en culture, dans un milieu de culture approprié, d'un hôte cellulaire préalablement transformé par un vecteur contenant un acide nucléique selon l'invention comprenant la séquence nucléotidique codant pour ledit polypeptide, et
- la récupération, à partir du susdit milieu de culture du polypeptide produit par ledit hôte cellulaire transformé.

A titre d'exemple d'amorces d'ADN ou d'ARN selon l'invention, on citera les couples de séquences suivants :
S1 : GTGATGAAGTTTTTAATGAATTATTAAA (SEQ ID n° 4)
S2 : TGTTGTTCTTGTTGTTGAACTTTTTACTAA (SEQ ID n° 5)
dont les positions respectives sur le gène LSA-3/K1 représente sur la figure 1 sont de 695 à 722 et de 829 à 799 (en lecture inverse), ou le couple:
6.1 : GGTATCGAAACTGAGGAAATAAAGG (SEQ ID n° 6)
6.2 : CATAGCAGGAACATCAACATCCAC (SEQ ID n°7)
dont les positions respectives sont 2668 à 2692 pour 6.1. et 3456 à 3433 pour 6.2. (lecture inverse).

Les informations sur les séquences ID n°4, ID n° 5, ID n° 6 et ID n° 7 sont détaillées à la fin de la description.

Les peptides d'intérêt selon la demande peuvent également être préparés par les techniques classiques de la synthèse de peptides. Cette synthèse peut être réalisée en solution homogène ou en phase solide. Par exemple, on aura recours à la technique de synthèse en solution homogène décrit par HOUBENWEYL dans l'ouvrage intitulé « Meuthode der Organischen Chemie » (Méthode de la Chimie Organique édité par E. Wunsch, vol. 15-I et II. THIEME, Stuttgart 1974 ou celle décrite par R..D. MERRIFIELD dans l'article intitulé « Solid phase peptide synthesis » (J. Am. Chem. Soc., 45, 2149-2154).

La demande décrit également sur les oligomères hydrosolubles des peptides monomères sus-indiqués.

L'oligomérisation peut provoquer un accroissement de l'immunogénicité des peptides monomères selon la demande. Sans qu'une telle indication chiffrée puisse être considérée comme limitative, on mentionnera néanmoins que ces oligomères peuvent, par exemple, contenir de 2 à 10 unités monomères.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode d'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro-bifonctionnels.

L'invention concerne encore les conjugués obtenus par couplage covalent des peptides selon l'invention (ou des susdits oligomères) à des molécules porteuses permettant notamment d'argumenter l'immunogénicité (naturelles ou synthétiques), physiologiquement acceptables et non toxiques, par l'intermédiaire de groupements réactifs complémentaires respectivement portés par la molécule porteuse et le peptide. A titre d'exemple de molécules porteuses ou supports macromoléculaires entrant dans la constitution des conjugués selon l'invention, on mentionnera des protéines naturelles, telles que l'anatoxine tétanique, l'ovalbumine, des sérums albumines, des hémocyamines, le PPD de la tuberculine (PPD : « Purified Protein Derivative »), etc...

A titre de supports macromoléculaires synthétiques, mon mentionnera par exemple des polylysines ou des poly(D-L-alanine)-poly(L-lysine).

A titre de supports hydrocarbones ou lipidiques, on mentionnera les acides gras, saturés ou insaturés, et préférentiellement ceux en C16 ou C18 de type oleyl ou palmitoleyl.

Enfin et sans être limitatifs, les antigènes ou peptides d'interêt selon la demande peuvent être couplés à des supports classiques ou adsorbées sur de tels supports notamment des microsphères ou billes de latex ou de polystyrène, ou incorporés dans des particules Ty1.

Pour synthétiser les conjugués selon l'invention, on peut avoir recours à des procédés connus en soi, tels que celui décrit par Frantz et Robertson dans Infect. and Immunity, 33, 193-198 (1981), ou celui décrit dans Applied and Environmental Microbiology, (octobre 1981), vol. 42, n° 4, 611-614 par P.E. Kauffman en utilisant le peptide et la molécule porteuse appropriée.

Les acides nucléiques décrits ici peuvent être préparés soit par un procédé chimique, soit par d'autres procédés.

Un mode de préparation approprié des acides nucléiques comportant au maximum 200 nucléotides (ou 200 pb, lorsqu'il s'agit d'acides nucléiques bicaténaires) de l'invention comprend les étapes suivantes :
- la synthèse d'ADN en utilisant la méthode automatisée des β-cyanethylphosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un mode de préparation, par voie chimique, d'acides nucléiques de longueur supérieure à 200 nucléotides a déjà été été décrit dans WO 92/13884.

La demande décrit également des trousses de diagnostic qui comportent une ou plusieurs amorces d'amplification spécifiques du gène LSA-3 et permettant de détecter la présence du gène ou du mRNA chez un individu susceptible d'être infecté par P.falciparum.

L'invention porte aussi sur des compositions pharmaceutiques ou vaccinales dans lesquelles l'un au moins des produits selon l'invention se trouve associé à des excipients pharmaceutiquement acceptables, solides ou liquides, adaptés à la construction de formes orales, oculaires ou nasales, ou des excipients adaptés à la construction des formes d'administration rectale, ou encore avec des excipients gélatineux pour l'administration vaginale. Elle concerne aussi des compositions liquides isotoniques contenant l'un au moins des conjugués selon l'invention, adaptées à l'administration sur les muqueuses, notamment oculaires ou nasales ou pulmonaires.

Avantageusement, les compositions vaccinales selon l'invention contiennent en outre un véhicule, tel que la polyvinyl-pyrrolidone,facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthyl-cellulose, les hydroxydes et phosphates d'aluminium, la saponine ou tous autres adjuvants de ce type, bien connus de l'homme de l'art. Enfin, elles contiennent si besoin un adjuvant immunologique, notamment du type muramylpeptide.

La demande décrit aussi des compositions pharmaceutiques contenant à titre de substance active l'un au moins des anticorps polyclonaux ou monoclonaux définis précédemment en association avec un véhicule pharmaceutiquement acceptable.

La demande décrit enfin sur une méthode d'immunisation d'un individu susceptible d'être infecté par P. falciparum par injection d'une molécule peptidique ou d'un oligomère tel que décrit précédemment, seul ou en association avec d'autres types de molécules susceptibles de protéger ledit individu contre une infection ultérieure, la molécule polypeptidique ou antigénique ou les lipopeptides naturels ou recombinants sont, soit utilisés seuls, soit adsorbés ou couplés à des microsphères ou billes de latex ou de polystyrène.

Des caractéristiques supplémentaires de l'invention apparaîtront encore dans les exemples illustrés des figures qui suivent et montrent les caractéristiques particulières des molécules d'intérêt de la demande par rapport à d'autres antigènes du stade pré-érythrocytaire du parasite.
La figure 1 représente la séquence ID n° 1 d'ADN génomique de 6152 paires de bases du gène LSA-3, elle est issue du clone K1.2 lui-même issu d'un isolat thaïlandais.
La figure 2 représente la séquence ID n° 2 du cDNA et la séquence polypeptidique de l'antigène LSA-3. La séquence de DNA représente 5361 paires de bases.
La figure 3 représente la séquence ID n° 3 de la partie séquencée dans le clone parasitaire T9/96 (1890 paires de bases), la ligne du haut étant la séquence nucléotidique et la ligne du bas la séquence peptidique. Dans ce clone, la séquence CT1 est comprise entre les nucléotides 67 et 126, le fragment DG 679 proprement dit commençant au nucléotide 207. Le fragment 729 RE est compris entre les nucléotides 547 et 629.
La figure 4a représente schématiquement les positionnements relatifs des séquences répétées et non répétées, des introns et des exons dans les souches K1 et T9/96, les clones 679 et 729 étant issus de ce dernier.
La figure 4b représente le HCP (Hydrophobic Cluster Plot) de la séquence peptidique du clone DG729.
La figure 5 représente les quantités d'immunoglobulines produites dans le sérum de chimpanzé Nuria avant et après l'immunisation par différents peptides de LSA-3.
La figure 6 indique le titre en anticorps spécifique de différentes espèces de souris immunisées soit avec un peptide soit avec un lipopeptide correspondant.
La figure 7 montre l'inhibition de l'invasion des cellules hépatiques par les sporozoïtes par des sérums hyper-immuns obtenus après immunisation par différents peptides immuno-purifiés contre du LSA-3 entier.
La figure 8 représente la comparaison d'un antigène issu de LSA-3 avec deux autres antigènes sur l'immunité de type T.

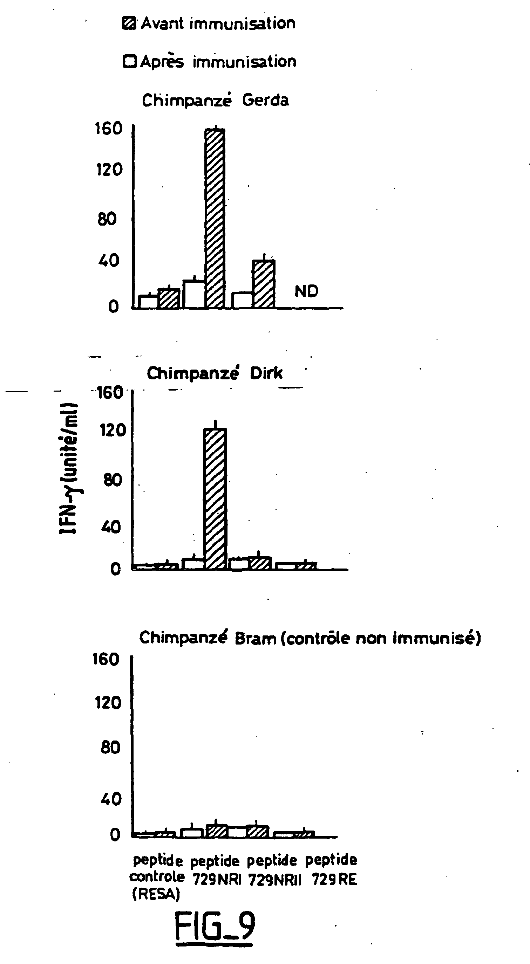

La figure 9 représente l'induction de l'interféron-γ chez les chimpanzés Gerda et Dirk par les peptides issus de la molécule LSA-3.
La figure 10 représente les résultats de lymphoprolifération des PBMC d'un individu protégé par injection de sporozoïtes irradiés contre des peptides issus des antigènes LSA-1 et LSA-3.

### Exemple 1 : Clonage et séquençage du gène LSA-3

### 1)séquençage

Le criblage initial de la génothèque provenant du clone parasitaire T9/96 avec le sérum d'un missionnaire soigné de façon continue en prophylaxie nous a permis d'isoler 120 clones correspondant à des molécules exprimées au stade sporozoïque et/ou hépatique du cycle de P.falciparum. Le clone 729S a été utilisé comme sonde pour cribler une banque génomique de la souche K1 thaïlandaise déjà citée plus haut qui contient de grands fragment Eco R1 clonés dans le phage lambda gt10. Un insert de 6,85 kilobases contenant le gène entier a été purifié de cette génothèque et recloné dans un plasmide pUC18 pour séquençage et caractérisation. Chez P.falciparum dont le génome est très riche en base A :T (80%), cette approche est souvent rendue difficile par la rareté des sites de restriction utilisables et par l'instabilité ou même l'impossibilité de cloner certains fragments lorsqu'ils sont insérés en vecteurs plasmidiques.

La structure du gène est représentée dans la figure 4 et présente les caractéristiques suivantes :
a) un mini exon 1 codant à son extrémité 3' pour un peptide signal hydrophobe;
b) un court intron (168 paires de bases) inclus entre des sites consensus de donneurs et accepteurs de splicing ;
c) un deuxième exon de cinq kilobases qui code pour une région organisée de 1,8 kilobases et composé d'un arrangement de 7 blocs de 4 amino acides, et d'une région hydrophobe en 3' qui pourrait correspondre à un encrage du type glycosyl-phosphatidyl-inositol (GPI).

Une investigation détaillée du polymorphisme du LSA-3 a été réalisée en séquençant le clone 679 qui contient l'essentiel des séquences répétées du gène LSA-3 et une portion de 1 kilobase de la fraction non répétée en 3', la séquence de ce fragment étant représentée dans la figure 3 entre les nucléotides 207 et 1890.

Les répétitions de la souche K1 sont les suivantes :
Bloc 1 : (aa223) VEEK VEES VEEN DEES VEEN VEEN VEEN DDGS VASS VEES IASS VDES IDSS IEEN (aa278)
Bloc 2 : (aa279) VAPT VEEIVAPS WESVAPS VEESVEEN VEESVAEN VEESVAEN VEESVAEN VEESVAEN VEEIVAPT VEEIVAPT VEEIVAPS WESVAPS VEESVEEN VEESVAEN VEESVAEN VEESVAEN VEESVAEN VEESVAEN VEEIVAPT VEEIVAPT VEEIVAPS WESVAPS VEESVEEN VEESVAEN VEESVAEN VEESVAEN VEESVAEN VEESVAEN VEESVAEN VEESVAEN VEEIVAPT VEEIVAPT VEEIVAPS WESVAPS VEESVEEN VEESVAEN VEESVAEN VEESVAEN VEESVAEN VEEIVAPT VEEIVAPT VEEIVAPS WESVAPS VEESVEEN VEESVAEN VEESVAEN VEESVAEN VEEIVAPT VEEIVAPT VEEIVAPS WESVAPS VEESVEEN VEESVAEN VEESVAEN VEESVAEN VEESVAEN VEEIVAPT VEEIVAPT VEEIVAPS WESVAPS VEESVEEN VEESVAEN VEESVAEN VEESVAEN VEESVAPT VEEIVAPS VEESVAPS VEESVAEN (ara818)
Bloc 3 :
   (aa1537)DEDI EEDV EEDI EEDI EEDK VEDI DEDI DEDI GEDK DEVI (aa1576)

Les répétitions dans le clone T9/96 telles que déterminées dans la demande de brevet n° FR 9101286 du 05 février 1991, sont les suivantes :
Bloc 1 : VEEK VEES VEEN DEES VEEN VEEN VEEN DDGS VASS VEES IASS VDES IDSS IEEN
Bloc 2 : VAPT VEEIVAPT VEEIVAPS VVESVAPS VEESVAPS VEESVAEN VEESVAEN VEEIVAPS VEESVAEN VEESVAEN VEESVAEN VEESVAEN VEESVAEN VEEIVAPT VEESVAPT VEEIVAPT VEESVAPT VEEIVVPS VEESVAPS VEESVAEN VEESVAEN VEESVAEN VEESVAEN VEESVAEN VEEIVAPS VEEIVAPT VEESVAEN

L'exon 2 du gène LSA3 comporte 2 régions répétées pouvant être décomposées en 3 blocs comme indiqué sur la figure 4 :
- le bloc 1, codant pour un enchaînement de 14 tétrapeptides. Ce bloc est conservé à 100% en acides aminés et en acides nucléiques entre T9/96 et K1. Seuls les tétrapeptides VEES et VEEN sont retrouvés dans le bloc 2.
- le bloc 2 code, dans K1, pour 127 tétrapeptides correspondant à l'enchaînement de différents octapeptides, eux-mêmes formés par combinaison de 2 des 7 tétrapeptides ou motifs de base (VEES, WES, VEEN, VEEI, VAEN, VAPS, VAPT). Le nombre de répétitions et l'arrangement de ces octapeptides varient selon les motifs et semblent être spécifiques du clone K1.2. En effet, dans le clone T9/96, le bloc 2 (53 tétrapeptides) correspond également à l'enchaînement d'octapeptides formés à partir des 7 mêmes tétrapeptides de base (et d'un 8ème motif VVPS n'existant pas dans K1), mais avec un nombre et un arrangement différents de ces répétitions.
- le bloc 3 est constitué de l'enchaînement de 10 tétrapeptides dégénérés et différents de ceux des blocs 1 et 2. Ce bloc a été séquencé uniquement dans la souche K1. Cependant, des résultats préliminaires obtenus par PCR avec le clone T9/96 et plusieurs autres souches de laboratoire indiquent qu'il n'existe pas de polymorphisme de taille dans cette région.

Les régions non-répétées de l'exon 2 sont particulièrement bien conservées. En effet, la comparaison de séquence entre T9/96 et K1 a pu être faite sur 315 bp en 5' du bloc 1 et sur 763 bp en 3' du bloc 2. L'homologie est de 99,4% en acides nucléiques et 98,6% en acides aminés.

La comparaison des séquences du clone 679 issu du clone de P.falciparum T9/96, et de la séquence correspondante du LSA-3 issue de l'isolat K1 montre que le gène est bien conservé, les différences les plus notables étant observées dans la région répétée où les blocs de 4 amino acides sont bien conservés mais varient dans leur nombre et leur organisation.

Au contraire les parties 5' et 3' non répétées apparaissent comme particulièrement bien conservées montrant jusqu'à 100% d'homologie dans la région 5' où des épitopes B et T ont déjà été identifiés.

Des amplifications d'ADN, notamment par PCR de différentes souches de P.falciparum avec 8 paires d'amorces réparties sur l'ensemble du gène LSA-3 ont montré que, excepté avec celles entourant les régions répétées, tout le génome donne des produits PCR de taille similaire ce qui suggère que l'antigène LSA-3 est bien conservé.

Diverse sondes LSA-3, choisies dans les régions répétées et non répétées, ont été hybridées à faible stringence avec les ADN de différentes espèces de Plasmodium et n'ont permis d'identifier aucun gène homologue à LSA-3, sauf chez le parasite de chimpanzé P.reichenowi, ce qui confirme la proche parenté de cette espèce avec P.falciparum.

De façon surprenante, l'antigène analogue à LSA-3 trouvé dans P. voelii, qui donne clairement des réactions immunologiques croisées à la surface du sporozoïte avec des anticorps contre le fragment 729S, ne semble pas être conservé au niveau de la séquence nucléotidique. Enfin la comparaison des séquences LSA-3 avec les bases de données n'a révélé aucune homologie avec des molécules connues, à l'exception de la région répétée dont certains motifs présentent une forte analogie avec les répétitions d'un gène de Staphilococus xylosis, mais aussi avec deux antigènes de P.falciparum, RESA et Pf11.1, tous deux exprimés au cours du stade sanguin du parasite. Cette homologie est essentiellement due à la richesse en séquences « glu-glu » de ces antigènes et des répétitions de LSA-3.

### 2) Clonage :

L'insert DG729 et d'autres régions de l'exon 2 de la souche K1 ont été clonés dans un vecteur d'expression procaryotique pGEX, vecteur commercialisé par la société In Vitrogene Corp (San Diego USA). Ce vecteur produit une protéine de fusion avec la glutathion -S-transferase (GST) de Schistosoma mansoni et permet une purification facile des protéines recombinantes par affinité du billes de glutathion-agarose. Les peptides d'expression à partir de ces vecteurs sont nommés :
- pour la protéine LSA-3 entière : protéine REC,
- ou pour le fragment 729S : 729PGEX.

Les tentatives de clonage d'autres fragments notamment le fragment 1-5 3NSREP, 3NFREP, 5NR, et 5SNREP ont posé des difficultés concernant soit le clonage soit la production et la purification des protéines en quantité suffisante pour des expériences d'immunisation.

Seuls les fragments 729, NN et 3PC ont permis de produire et de purifier des polypeptides recombinants correspondants en quantité suffisante pour l'analyse de l'antigénicité de la molécule.

### Exemple 2: Protection de chimpanzés immunisés contre des injections d'épreuve à faible ou forte dose :

2.1. Un chimpanzé Dirk préalablement immunisé par une fraction de LSA-3 en combinaison avec un autre antigène du même stade de développement du parasite, et présentant les effets décrits plus haut au point a), a été re-immunisé quelques années plus tard par des peptides et des protéines recombinantes correspondant à la même combinaison d'antigènes. De nouveau, ce chimpanzé s'avère protégé contre une infection d'épreuve à faible dose (2.10⁴ sporozoïtes) puis une infection d'épreuve à forte dose (5.10⁶ sporozoïtes). Comme au cours du premier challenge, on observe une réduction importante du nombre de schizontes détectés dans le foie après le challenge à forte dose ainsi qu'un infiltrat lympho-monocytaire autour des rares schizontes détectables (témoin d'une défense locale).

2.2. Protection partielle du chimpanzé Gerda : un autre chimpanzé a été immunisé uniquement par l'antigène LSA-3 (animal décrit dans les exemples 7 et 8 ci-après), à savoir le lipopeptide NR2 puis les protéines recombinantes (GST-729, GST-NN, GST-3PC) qui couvrent à elles trois 95 % de la molécule LSA-3 et adsorbées sur microsphères de latex. Cet animal s'avère être partiellement protégé contre une infection d'épreuve à forte dose (8.10⁶ sporozoïtes) puisqu'il présente une très faible parasitémie sanguine et une réduction de 90 % du nombre de schizontes hépatiques par rapport au contrôle suite à l'infection d'épreuve.

2.3 Protection partielle du chimpanzé Nuria : un chimpanzé immunisé par une fraction de l'antigène LSA-3 seul, à savoir une combinaison de peptides, de lipopeptides puis de protéines recombinantes correspondant à 95 % de la molécule LSA-3 et émulsifiées dans le Montanide ISA-51 (SEPPIC, 75 Quai d'Orsay, France), s'avère être partiellement protégé contre une infection d'épreuve à dose moyenne (1.10⁵ sporozoïtes). En effet, cet animal présente un retard significatif de l'apparition des parasites dans le sang par rapport à 4 témoins (chimpanzés immunisés par les antigènes pré-érythrocytaires LSA-1, SALSA ou STARP, et 1 animal témoin non immunisé), une parasitémie sanguine maxima plus faible et une chute de parasitémie plus rapide (24 heures au lieu de 3 jours), résultats qui traduisent une forte réduction du nombre de formes hépatiques induites chez cet animal par l'infection d'épreuve et en accord avec les résultats obtenus chez Gerda. Dans ce cas, l'examen des formes hépatiques n'a pas été réalisé.

2.4 Immunogénicité B. T chez les chimpanzés Demi, Karlien et Iris :Trois chimpanzés immunisés par les peptides LSA-3- NRI et -RE et les lipopeptides - NRII et -CT1, ainsi que par des peptides correspondant, pour chaque animal, à un autre antigène pré-érythrocytaire (LSA-1, SALSA ou STARP) présentent tous les 3:
- des réponses humorales élevées contre les épitopes B présents sur les peptides NRI NRII et RE. Les anticorps reconnaissent non seulement les peptides et les recombinants mais sont aussi fortement positifs sur les molécules natives du parasite, ce qui est apprécié par immunofluorescence sur les sporozoïtes et les stades hépatiques de Plasmosdium falciparum (mais négatifs vis-à-vis des stades érythrocytaires) ;
- des réponses lymphoprolifératives élevées et spécifiques contre les 4 peptides LSA-3 ainsi que les épitopes T natifs présents à la surface des sporozoïtes de Plasmodium falciparum et de Plasmodium yoelii, chez lequel LSA-3 possède un homologue (non encore caractérisé).

Les réponses B et T vis-à-vis des antigènes natifs sont un point important car:
a) elles prouvent la bonne représentativité des molécules synthétiques ;
b) elles signifient qu'au moment de l'infection, il y a de bonnes chances d'obtenir une réponse secondaire anamnestique ; c'est de fait ce qui a été observé chez le chimpanzé Nuria au moment du challenge. L'importance de cette observation est confortée par le fait que la même réponse secondaire n'a pas été obtenue vis-à-vis des autres antigènes tels que LSA-1 et STARP.

2.5. Immunogénicité chez l'Aotus : un singe-hibou (Aotus trivirgatus) immunisé par les 2 peptides LSA-3- NRI et -RE et les 2 lipopeptides - NRII et -CT1, puis re-stimulé par les protéines recombinantes correspondant à 95 % de la molécule LSA-3 et adsorbées sur microsphères comme décrit ci-dessus, présente des réponses lymphoprolifératives élevées et spécifiques contre les épitopes T présents sur ces mêmes peptides.

Pour ce qui est de la réponse in vivo des différents chimpanzés ainsi pré-immunisés, les résultats soulignent l'excellente immunogénicité (B et T) de LSA-3, sous forme peptide, lipopeptide et recombinants, et dans tous les modèles animaux testés jusqu'à présent, soit 6/6 chimpanzés (outbred), 1/1 aotus, et chez toutes les souris immunisées (>20). Notons que les résultats des formulations lipopeptidiques (qui peuvent être utilisées chez l'Homme) ont été obtenus par injections sous-cutanées en l'absence de tout adjuvant.

### Exemple 3 : Identification d'épitope CTL

La méthode utilisée pour identifier les CTL est celle décrite dans Fidock et al, (1994), J. Immunol. 153 : 190 ou dans Bottius et al, (1996), J. Immunol 156 :2874-2884.

Des épitopes CTL (pour lymphocytes T cytotoxiques) ont été identifiés dans les peptides NRII, RE et CT1 grâce à des tests de cytotoxicité effectués à partir des PBMC des chimpanzés Dirk, Gerda, Nuria, Demi, Karlien et Iris décrits plus haut.

Chez l'homme, 8 épitopes CTL additionnels, dont 7 situés dans la région 3' non-répétée, ont pu être mis en évidence à partir des PBMC d'individus appartenant à 3 haplotypes différents (MHC classe 1-A2, -B8 et -B53) et vivant en région d'endémie (Gambie) (résultats non publiés). De plus, le séquençage des 2 épitopes CTL restreints par B53 a démontré une parfaite conservation de leurs séquences nucléotidique et peptidique dans plusieurs souches du Kenya et de Gambie.

Au total, nous avons identifié 11 épitopes CTL dans la molécule LSA-3, ce qui est considérable. Par ailleurs, 5 chimpanzés/6 ont développé des réponses CTL contre le peptide NRII après immunisation par le lipopeptide NRII sans adjuvant, ce qui est un résultat remarquable pour des animaux non-consanguins. D'autre part, dans la mesure où les anticorps développés par Nuria ne présentaient aucune activité inhibitrice de l'invasion des sporozoïtes de Plasmodium falciparum, on peut supposer que la protection observée dépendait des réponses cellulaires, en particulier des CTL.

### Exemple 4 : Comparaison des titres anticorps avant ou après immunisation par différents peptides

4.1. Comparaison des réponses en anticorps induites par différents peptides dans différents animaux immunisés.

La méthode utilisée est celle décrite dans Behr et al, (1992), J. Immunol 149 : 3321.

La réactivité est exprimée en ratio Elisa, c'est-à-dire la densité optique mesurée à 496 nanomètres du sérum après immunisation rapporté à la densité optique du même sérum avant immunisation. La première colonne indique l'animal immunisé, la deuxième colonne l'immunogène reçu par l'animal, la 3ème colonne indique le nombre d'injections réalisées ainsi que le support accompagnant le peptide injecté : RP signifie protéine recombinante, RP/B signifie protéine recombinante adsorbée sur billes de latex, P signifie peptide et LP lipopeptide. Il faut préciser en outre que les lipopeptides sont injectés dans du sérum physiologique, les peptides et les protéines recombinantes sont adsorbés sur les billes de latex ou dans une émulsion avec un adjuvant montanide ISA-51.

**tableau I : REACTIVITE ANTICORPS DES DIFFERENTS PEPTIDES EXPRIMEE EN RATIO ELISA**

| | | | LSA1 | | | | SALSA | | STARP | | LSA3 | | | | |
|---|---|---|---|---|---|---|---|---|---|---|---|---|---|---|---|
| Chimpanzés | Immunogène | nbre et type d'injection | LSA-REP | LSA-J | LSA -NR | LSA-TER | SALSA -1 | SALSA -2 | STARP-M | STARP-R | LSA 3 -CT1 | LSA 3 NR1 | LSA 3-NR II | LSA 3-REP | R32T et 32 |
| Animaux immunisés | | | | | | | | | | | | | | | |
| DIRK | LSA3 et | 3RP(d) | 7.4 | 9.0 | 0.9 | 0.8 | nd | nd | 0.5 | 0.7 | 1.7 | 1.0 | 1.1 | 8.8 | 0.7 |
| | LSA1 | 3RP + 3 (P + LP) | 20.0 | 10.0 | 0.1 | 0.4 | 0.2 | 1.1 | 1.0 | 0.6 | 1.0 | 1.1 | 3.1 | 17.0 | 0.8 |
| GERDA | LSA3 | 3LP | nd | nd | nd | nd | nd | nd | nd | nd | nd | nd | 3.9 | nd | 0.6 |
| | | 3LP + 3 RP/B | nd | nd | nd | nd | nd | nd | nd | nd | 0.7 | 1.1 | 3.0 | 12.3 | 0.9 |
| DEMI | LSA3 et | 2 (P+LP) | 8.0 | 14.1 | 0.7 | 16.4 | 0.6 | 1.1 | nd | nd | 0.7 | 1.5 | 11.7 | 19.1 | 0.7 |
| | LSA1 | 3 (P+LP) | 8.4 | 14.5 | 1.8 | 21.5 | 0.8 | 0.2 | nd | nd | 0.8 | 5.1 | 14.2 | 20.7 | 1.2 |
| | | 3 (P+LP)+ 3RP/B | | | | | | | | | | | | | |
| KAR- | LSA3 et | 2 (P+LP) | 0.5 | 1.2 | 1.0 | 1.0 | 1.1 | 2.1 | nd | nd | 1.0 | 3.6 | 3.1 | 10.3 | 0.9 |
| LIEN | SALSA | 3 (P+LP) | 1.1 | 0.2 | 0.5 | 0.2 | 1.8 | 2.5 | nd | nd | 1.4 | 4.7 | 6.8 | 14.1 | 0.6 |
| | | 3 (P+LP) + 3 RP/B | | | | | | | | | | | | | |
| IRIS | LSA3 et | 2 (P+LP) | nd | nd | nd | nd | nd | nd | 10.1 | 15.9 | 0.7 | 2.4 | 6.7 | 12.5 | 0.6 |
| | STARP | 3 (P+LP) | nd | nd | nd | nd | nd | nd | 10.5 | 16.4 | 1.3 | 3.1 | 6.8 | 15.3 | 0.5 |
| | | 3 (P+LP) + 3 RP/B | | | | | | | | | | | | | |

| Contrôles non immunisés : | | | | | | | | | | | | | | | |
|---|---|---|---|---|---|---|---|---|---|---|---|---|---|---|---|
| COR | β.GAL | 3 RP | 0.6 | 0.7 | 0.8 | 0.9 | 0.5 | 1.0 | 1.2 | 0.8 | 1.1 | 1.0 | 0.6 | 1.1 | 1.2 |
| PEER | β-GAL | 8 RP | 1.1 | 0.8 | 0.7 | 0.9 | 0.8 | 1.2 | 1.0 | 0.9 | 1.1 | 0.6 | 0.9 | 0.9 | 0.3 |
| BRAM | GST | 2 RP | 1.1 | 0.6 | 0.5 | 1.1 | 0.3 | 0.8 | 0.9 | 1.2 | 1.1 | 0.3 | 0.4 | 0.7 | 1.0 |
| | | 3 RP | 0.8 | 0.3 | 0.8 | 1.3 | 0.7 | 1.2 | 1.1 | 1.2 | 1.6 | 0.2 | 1.3 | 0.6 | 0.4 |
| FOUAD | PBS | | 0.9 | 0.5 | 1.0 | 0.6 | 0.8 | 1.3 | 1.0 | 0.3 | 1.9 | 1.3 | 0.3 | 0.2 | 0.9 |

4.2. Titre des anticorps obtenus :
Le tableau 2 représente les titres d'anticorps des sérums obtenus chez les chimpanzés par immunofluorescence sur les antigènes natifs présents à la surface des différents stades (sporozoïques, hépatiques et sanguins) de P. falciparum, P. yoelii et P. berghei.

**tableau II : TITRE EN ANTICORPS IMMUNOFLUORESCENTS**

| | | | P. falciparum | | P. yoelii | (17XL et 17XNL) | |
|---|---|---|---|---|---|---|---|
| CHIMPANZE | Antigène | SS (NF54) | LS (NF54 et 73OXI) | BS (150) | SS | LS | BS |
| Animaux | immunisés | | | | | | |
| DIRK | LSA3 et LSA1 | 800 | 200 | -(<100) | 200 | 200 | -(<100) |
| GERDA | LSA3 | 400 | 200 | -(<100) | 400 | 200 | -(<100) |
| DEMI | LSA3 et LSA1 | 100 | 400 | -(<100) | | | |
| KARLIEN | LSA3 et SALSA | 100 | 200 | -(<100) | | | |
| IRIS | LSA3 et STARP | 400 | 100 | -(<100) | | | |

| Animaux | témoins | | | | | | |
|---|---|---|---|---|---|---|---|
| COR | β-GAL | -(<100) | -(<100) | -(<100) | -(<100) | -(<100) | -(<100) |
| BRAM | GST | -(<100) | -(<100) | -(<100) | -(<100) | -(<100) | -(<100) |
| FOUAD | PBS | -(<100) | -(<100) | -(<100) | -(<100) | -(<100) | -(<100) |

4.3 Réponse lymphoproliférative des PBMC des différents chimpanzés après stimulation in vitro soit par les différents peptides soit par les antigènes natifs présents à la surface des sporozoïtes. Cette réponse a été mesurée par incorporation de thymidine tritiée dans des PBMC (cellules de sang périphériques) soit après stimulation par les peptides LSA-3 (tableau III) ou après stimulation in vitro avec des sporozoïtes (tableau IV).

**tableau III : INCORPORATION DE THYMIDINE TRITIEE DANS DES PBMC APRES STIMULATION PAR LES PEPTIDES LSA-3**

| Chimpanzé | Immunogène | LSA3-CT1 | LSA3-NRI | LSA3-NRII | LSA3-REP | MSP3-C (a) | PPPD(b) |
|---|---|---|---|---|---|---|---|
| Animaux | Immunisés | | | | | | |
| DIRK | LSA3 et LSA1 | 94 256 | 27 125 | 32 455 | 69 321 | 796 | 89 338 |
| | | (4.0) | (8.5) | (10.7) | (32.3) | (1.0) | (50.3) |
| GERDA | LSA3 | 13 359 | 1 429 | 13 236 | 14 883 | 485 | 29 355 |
| | | (25.1) | (2.8) | (25.6) | (28.6) | (0.9) | (132.3) |
| DEMI | LSA3 et LSA1 | 30 036 | 17 221 | 4178 | 52 301 | 689 | 167 277 |
| | | (46.8) | (27.4) | (7.3) | (81.2) | (1.1) | (113.3) |
| KARLIEN | LSA3 et SALSA | 30 025 | 10 039 | 18 365 | 31 312 | 575 | 96212 |
| | | (36.4) | (12.8) | (23.1) | (38.0) | (0.7) | (82.3) |
| IRIS | LSA3 et STARP | 53 312 | 25 223 | 6 458 | 35 078 | 799 | 196 223 |
| | | (62.6) | (34.8) | (9.7) | (47.5) | (0.9) | (62.3) |

| Animaux non | immunisés | | | | | | |
|---|---|---|---|---|---|---|---|
| COR | β-GAL | 1399 | 2599 | 3625 | 788 | 2600 | 19 395 |
| | | (0.6) | (1.0) | (1.3) | (0.3) | (1.1) | (22.3) |
| PEER | β-GAL | 1225 | 1 389 | 3251 | 2960 | 3 962 | 59 399 |
| | | (0.2) | (0.3) | (1.2) | (0.9) | (1.5) | (22.3) |
| BRAM | GST | 1201 | 509 | 2501 | 2659 | 2 745 | 39 399 |
| | | (0.4) | (0.2) | (0.7) | (0.6) | (0.7) | (22.3) |
| FOUAD | PBS | 1211 | 1.310 | 956 | 688 | 655 | 136258 |
| | | (1.2) | (1.3) | (0.9) | (0.6) | (0.5) | (82.3) |

| | | | | | | | |
|---|---|---|---|---|---|---|---|
| a) Peptide de contrôle à partir de l'antigène MSP3 dans le sang b) PPD = Dérivé de protéine purifié à partir de Mycobacterium turberculosis. | | | | | | | |

**tableau IV: INCORPORATION DE THYMIDINE TRITIEE DANS DES PBMC APRES STIMULATION IN VITRO AVEC DES SPOROZOITES.**

| | | P. falciparum sporozoïtes | P. yoellii sporozoïtes | P. berghei sporozoïtes |
|---|---|---|---|---|
| Chimpanzé | Antigène | | | |

| animaux immunisés | | | | |
|---|---|---|---|---|
| DIRK | LSA3 et LSA1 | 10 402 (12.1) | 5 552 (5.6) | 2110 (2.0) |
| GERDA | LSA3 | 24 021 (20.5) | 18 228 (18.6) | 2 430 (0.7) |
| DEMI | LSA3 et LSA1 | 2111 (3.2) | 935 (1.4) | 214 (0.1) |
| KARLIEN | LSA3etSALSA | 4 402 (8,5) | 2 228 (3.8) | 914 (2.1) |
| IRIS | LSA3etSTARP | 9 816 (14.2) | 5 304 (8.1) | 614 (2.0) |

| animaux témoins | | | | |
|---|---|---|---|---|
| BRAM | GST | 245 (0.4) | 1 295 (1.8) | 514 (1.2) |
| FOUAD | PBS | 997 (1.5) | 828 (1.8) | 714 (1.1) |

Les réponses lymphoprolifératives sont indiquées en différence de comptage en coups par minute (Δ CPM) entre le nombre de coups obtenus en présence d'antigène diminué du nombre de coups en l'absence d'antigène. Les chiffres entre parenthèses indiquent les indices de stimulation, c'est-à-dire le rapport du nombre de coups obtenus en présence d'antigènes sur le nombre de coups obtenus en l'absence d'antigènes.

Les résultats sont considérés comme positifs quand le Δ CPM est supérieur à 1000 et quand l'indice de stimulation est supérieur à 3.

4.4. Comparaison des réponses en anticorps de chimpanzés Nuria avant et après immunisation par différents peptides.

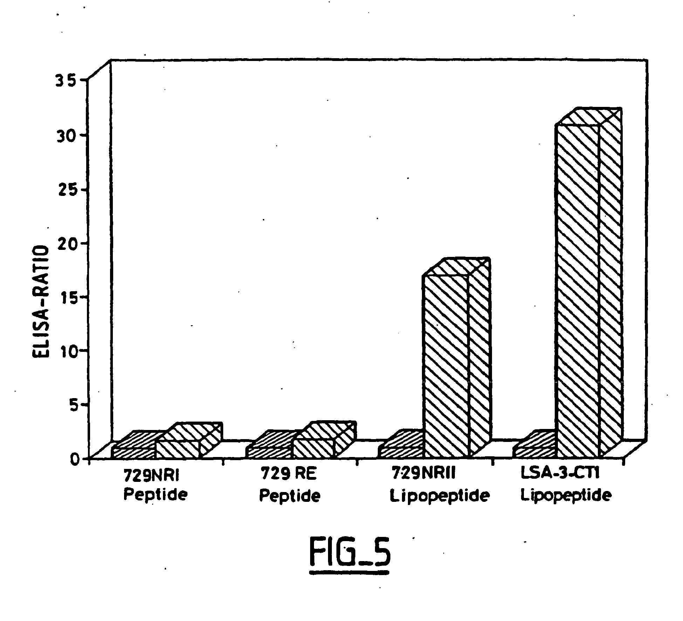

La figure 5 représente les quantités d'immunoglobulines présentes dans le sérum des chimpanzés Nuria avant et après immunisation par les peptides 729 NRI et 729RE, et les lipopeptides 729 NRII, et CT1.

Cette expérience montre la supériorité quant à l'immunité B de l'antigène R, surtout quand il est conjugué à un résidu lipidique.

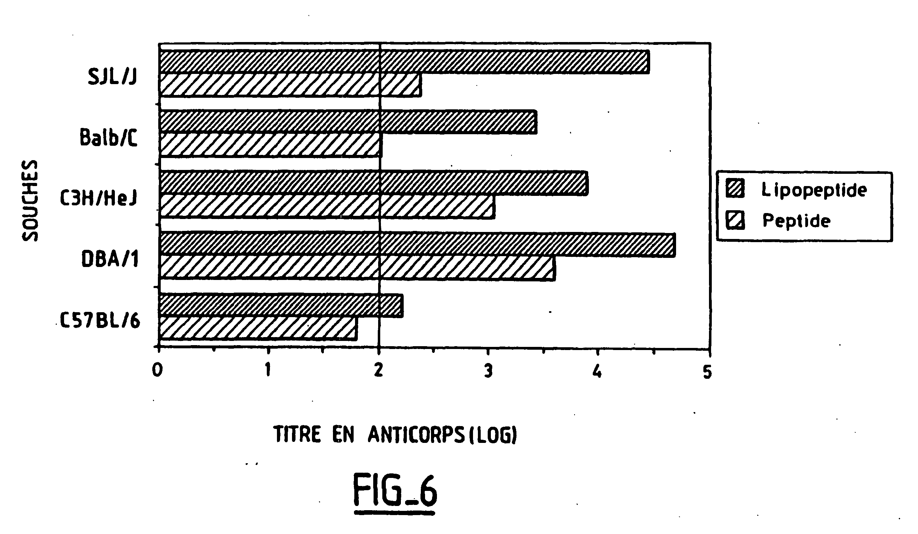

La figure 6 montre que le niveau d'anticorps spécifiques mesuré par Elisa contre le peptide 729 NRII dans des souris immunisées avec soit le peptide 729 NRII soit le lipopeptide 729NRII est nettement supérieur quand le lipopeptide est utilisé quelle que soit l'espèce de souris.

Exemple 5 : Lymphoprolifération des PBMC d'un individu protégé par injection de sporozoïtes irradiés contre des peptides issus des antigènes LSA-1 et LSA-3.

Chez huit volontaires humains immunisés par injection de sporozoïtes irradiés, des anticorps anti-LSA-3 sont trouvés chez chacun des quatre individus résistants à une infection par sporozoïtes; aucun chez les quatre autres volontaires ayant développé une infection sanguine.

De plus, pour le seul de ces quatre individus protégés dont les cellules étaient accessibles, les PBMC ont été prélevées six mois après l'infection d'épreuve et incubées en présence des peptides issus des antigènes LSA-1 et LSA-3.

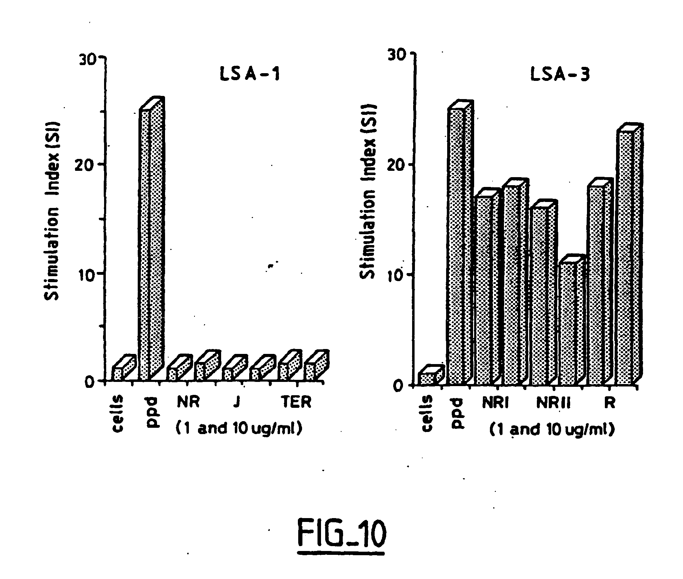

La figure 10 représente les résultats des lymphoproliférations des PBMC d'un individu protégé par injection des sporozoïtes irradiés contre des peptides issus des antigènes LSA-1 et LSA-3.

Des lymphoproliférations importantes ont été observées avec chacun des trois peptides LSA-3 ( NRI, NRII et RE) mais avec aucun des peptides LSA-1. Il existait un niveau particulièrement élevé de sécrétion d'IFN-γ (100 Ul/ml) après stimulation par le peptide NRI et, à un moindre degré, par le peptide NR2 (IFN-γ : la cytokine ayant le plus fort effet bloquant sur la schizogonie hépatique).

### Exemple 6 : Effets des anticorps contre les peptides de LSA-3 sur l'inhibition de la pénétration des sporozoïtes chez la souris.

Les techniques utilisées pour préparer les cultures primaires d'hépatocytes, les sporozoïtes, les anticorps et le test de fluorescence indirecte sont décrites en détail par S.MELLOUK et al, Bulletin of the World Health Organization, 68 : 52-59, 1990. Le tableau V ci-dessous compare les résultats obtenus en immuno fluorescence soit par des anticorps contre le fragment 679 soit par des anticorps obtenus contre des fragments en provenance d'autres peptides. La colonne de gauche indique le nombre de schizontes détectés après 48h de culture dans des hépatocytes de souris Balb c infectées par P.yoelii et la colonne de droite les mêmes paramètres après infection par P.Berghei.

**Tableau V:**

| Clones d'anticorps | P.yoelii | | | P.berghei | | |
|---|---|---|---|---|---|---|
| | IFA | Nbre de LS à 48 h | | IFA | Nbre de LSà48h | |
| | | a) | b) | | | |
| Contrôle | | 88 | 110 | | 119 | 108 |
| 679 | ++ | | 0 | - | | 47 |
| | ++ | | 0 | - | | ND |
| 679 | ++ | 1 | | - | 105 | |
| 679b | ++ | 1 | | - | 133 | |
| 679c | ++ | 1 | | - | 30 | |
| | | | | | | |
| 32 | ++ | 8 | | ± | 103 | |
| 222 | + | | 5 | ± | | 26 |
| 667 | ++ | 276 | 143 | ND | 502 | |
| 362 | + | 3 | | | | |
| 493 | ++ | 55 | | ND | 508 | |
| | | | | | | |
| α P.b. CSP Mab | | | 82 | +++ | | 30 |
| α P.y. CSP Mab | +++ | | 171 | | 138 | |
| | | | | | | |

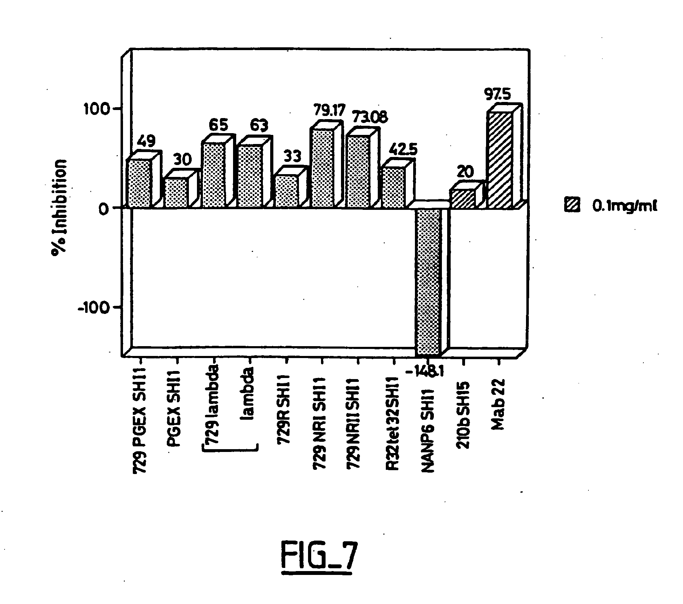

Il apparaît clairement que l'anticorps contre le peptide 679 a un effet d'inhibition quasi total du nombre de ce qu'ils ont observés à 48h dans les cellules hépatiques. De la même façon La figure 7 montre l'inhibition de l'invasion des cellules hépatiques par les sporozoïtes par des sérums hyper humains obtenus après immunisation par différents peptides et immuno purifiés contre du LSA-3 entier.

En ce qui concerne les protections de souris, les meilleurs résultats ont été obtenus par immunisation avec les recombinants, ou antigènes préparés selon la demande, adsorbés sur des microsphères de latex ou de polystyrène de 0,5µm de diamètre :
- 3/3 souris sont protégées contre une administration par 10 fois la dose minimale infectieuse
- 3/3 souris sont protégées contre le second challenge
- 2/3 souris sont protégées contre le troisième challenge.

Les microsphères utilisées sont les Polybead ® Polystyrène Microsphères (Polysciences, Inc.) de 0,50 µm de diamètre (réf.07307) sur lesquelles les recombinants ou les peptides sont adsorbés passivement. En pratique, chez la souris, pour 1 injection, 50µg d'antigènes sont mis en contact avec 50µl de microbilles ; la quantité exacte d'antigènes adsorbée n'est pas déterminée. Chez le chimpanzé, la même procédure est effectuée avec 200 µg d'antigènes et 200 µl de billes.

De plus, récemment, l'immunisation de souris par le recombinant GST-3PC (correspondant à la région 3' non-répétée de l'acide aminé n° 869 au codon stop en 3') a permis d'obtenir des sérums réagissant très fortement en immunofluorescence sur les sporozoïtes de Plasmodium falciparum. Ce résultat est la première démonstration de la présence d'un ou de plusieurs épitopes B dans cette région de la molécule.

### Exemple 7 : test de cytotoxicité contre le peptide 729 NRII chez le chimpanzé Gerda.

Le chimpanzé Gerda a été immunisé par voie i.v. avec le lipopeptide 729NRII issu de l'antigène LSA-3. Le sang est prélevé 9 jours après la 4ème injection. Les PBMC ont été incubés in vitro avec 5µg/ml du peptide 729NRII (ajout de l'IL2 recombinante, 10 U/ml, au jour 3). Au jour 15, l'activité cytotoxique a été étudiée contre les blastes autologues générés par la PHA à 0,5µg/ml. Les blastes ont été préincubés la nuit avec 5 µg/ml du peptide 729NRII, et avec un peptide contrôle : le RESA, ou sans peptide. Les peptides ne sont pas ajoutés pendant le test (8 heures). Le nombre de cibles par puits est de 5000.

Les PBMC de Gerda incubées pendant la même période avec 5µg/ml d'un peptide contrôle ou le peptide 729NRI (issu du même antigène), n'engendrent pas la lyse des blastes autologues préincubés ou non avec les peptides ci-dessus.

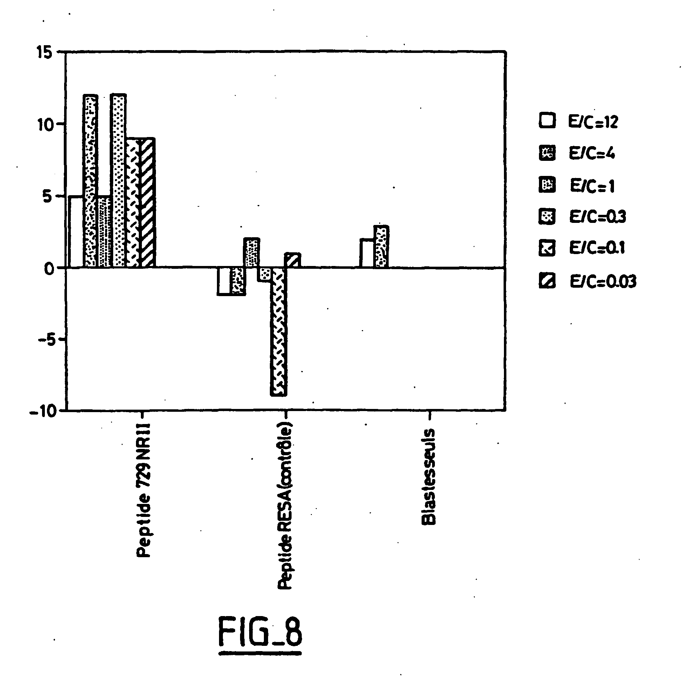

La figure 8 indique les résultats obtenus pour un rapport E/C (effecteur sur cible) variant de 12 à 0,03. On voit que les cellules cibles pré-sensibilisées par le peptide 729NRII sont lysés en présence de cellules effectrices ce qui indique une réponse immunitaire de type T cytotoxique spécifique de cet antigène.

Le lipopeptide NRII, injecté par voie IV, est capable, sans adjuvant, d'induire une réponse cytotoxique spécifique.

### Exemple 8 : effet du peptide NRI sur la production d'interféron γ.

Il a été montré que les interférons avaient une activité inhibitrice dans le développement du P.falciparum dans les hépatocytes humains en culture (Sylvie Mellouk et al, The Journal of Immunology, vol 139 n° 12 : 41-92, 41-95, 1987). Les résultats obtenus avec les peptides de l'invention sont les suivants :

Le chimpanzé Gerda, immunisé par la polypeptide NRII et boosté par le recombinant DG729, porte des PBMC capables de sécreter des taux élevés d'IFN-γ en présence des peptides LSA-3, en particulier le peptide 729 NR1. Le résultat a été confirmé chez le chimpanzé Dirk, immunisé par la même protéine. Le chimpanzé BRAM, contrôle non immunisé, ne montre aucune interféronémie contre les peptides LSA-3.

## Revendications

1. Conjugué **caractérisé en ce qu'**un résidu lipidique en C16 ou C18 est couplé par un pont lysine à un peptide de Plasmodium falciparum choisi parmi NRI, NRII, 729RE ou CT1.

2. Conjugué selon la revendication 1, **caractérisé en ce que** le résidu lipidique est saturé ou insaturé.

3. Conjugué selon la revendication 1 ou 2, **caractérisé en ce que** le résidu lipidique est un palmitoyle ou un palmityle ou un oléyle.

4. Conjugué immunogène dont la séquence polypeptidique est d'origine d'un isolat de *P*. *falciparum* et présente au moins 70% d'homologie avec celle d'un conjugué selon l'une des revendications 1 à 3.

5. Composition immunogène **caractérisée en ce qu'**elle contient au moins un conjugué selon l'une quelconque des revendications 1 à 4 et au moins un véhicule pharmaceutique.

6. Composition immunogène selon la revendication 5, **caractérisée en ce que** le véhicule pharmaceutique est la polyvinyl-pyrrolidone.

7. Composition immunogène selon la revendication 5, **caractérisée en ce que** la composition contient un adjuvant.

8. Composition immunogène selon la revendication 5, **caractérisée en ce que** l'adjuvant est la carboxyméthyl-cellulose, un hydroxyde ou un phosphate d'aluminum, la saponine ou un muramylpeptide.

9. Composition de vaccin anti-palustre, contenant entre autres principes immunogènes, un conjugué selon l'une des revendications 1 à 4.

10. Méthode de diagnostic *in vitro* du paludisme chez un individu susceptible d'être infecté par *P. falciparum* qui comprend la mise en contact d'un tissu ou d'un fluide biologique prélevé chez un individu, avec un conjugué selon l'une des revendications 1 à 4 dans des conditions permettant une réaction immunologique entre ledit conjugué et les anticorps éventuellement présents dans le tissu ou le fluide biologique, et la détection *in vitro* des complexes antigènes / anticorps éventuellement formés.

11. Utilisation d'un conjugué selon l'une des revendications 1 à 4 dans la préparation d'un vaccin anti-palustre.

## Claims

1. Conjugate, **characterised in that** a C16 or C18 lipid residue is coupled by a lysine bridge to a peptide of *Plasmodium falciparum* selected from among NRI, NRII, 729RE or CT1.

2. Conjugate according to claim 1, **characterised in that** the lipid residue is saturated or unsaturated.

3. Conjugate according to claim 1 or 2, **characterised in that** the lipid residue is a palmitoyl or a palmityl or an oleyl.

4. Immunogenic conjugate the polypeptide sequence of which originates from an isolate of *P. falciparum* and has at least 70% homology with that of a conjugate according to one of claims 1 to 3.

5. Immunogenic composition, **characterised in that** it contains at least one conjugate according to any one of claims 1 to 4 and at least one pharmaceutical vehicle.

6. Immunogenic composition according to claim 5, **characterised in that** the pharmaceutical vehicle is polyvinylpyrrolidone.

7. Immunogenic composition according to claim 5, **characterised in that** the composition contains an adjuvant.

8. Immunogenic composition according to claim 5, **characterised in that** the adjuvant is carboxymethylcellulose, a hydroxide or a phosphate of aluminium, saponine or a muramylpeptide.

9. Anti-malarial vaccine composition, containing among other immunogenic principles a conjugate according to one of claims 1 to 4.

10. Method of *in vitro* diagnosis of malaria in an individual likely to be infected with *P. falciparum,* which comprises bringing a tissue or biological fluid taken from an individual into contact with a conjugate according to one of claims 1 to 4, under conditions enabling an immunological reaction between said conjugate and any antibodies present in the tissue or the biological fluid, and detecting *in vitro* any antigen/antibody complexes that may be formed.

11. Use of a conjugate according to one of claims 1 to 4 in the preparation of an anti-malarial vaccine.

## Patentansprüche

1. Konjugat, **dadurch gekennzeichnet, dass** ein Lipidrest aus C 16 oder C 18 über eine Lysinbrücke mit einem Peptid aus Plasmodium falciparum, ausgewählt aus NRI, NRII, 729RE oder CT1, gekoppelt ist.

2. Konjugat nach Anspruch 1, **dadurch gekennzeichnet, dass** der Lipidrest gesättigt oder ungesättigt ist.

3. Konjugat nach Anspruch 1 oder 2, **dadurch gekennzeichnet, dass** der Lipidrest ein Palmitoyl oder ein Palmityl oder ein Oleyl ist.

4. Immunogenes Konjugat, dessen Polypeptidsequenz aus einem Isolat von *P. falciparum* stammt und mindestens 70% Homologie mit der eines Konjugats gemäß einem der Ansprüche 1 bis 3 aufweist.

5. Immunogene Zusammensetzung, **dadurch gekennzeichnet, dass** sie mindestens ein Konjugat gemäß einem beliebigen der Ansprüche 1 bis 4 und mindestens einen pharmazeutischen Träger umfasst.

6. Immunogene Zusammensetzung nach Anspruch 5, **dadurch gekennzeichnet, dass** der pharmazeutische Träger Polyvinylpyrrolidon ist.

7. Immunogene Zusammensetzung nach Anspruch 5, **dadurch gekennzeichnet, dass** die Zusammensetzung ein Adjuvans umfasst.

8. Immunogene Zusammensetzung nach Anspruch 5, **dadurch gekennzeichnet, dass** das Adjuvans Carboxymethyl-Zellulose, ein Hydroxid oder ein Aluminiumphosphat, Saponin oder ein Muramylpeptid ist.

9. Anti-Malaria-Impfstoffzusammensetzung, umfassend neben anderen immunogenen Bestandteilen ein Konjugat gemäß einem der Ansprüche 1 bis 4.

10. *In vitro*-Diagnostizierverfahren von Malaria bei einem Individuum, das empfänglich dafür ist, von *P. falciparum* infiziert zu werden, umfassend das In-Kontakt-Bringen eines Gewebes oder einer biologischen Flüssigkeit, die einem Individuum entnommen wurden, mit einem Konjugat gemäß einem der Ansprüche 1 bis 4 unter Bedingungen, die eine Immunreaktion zwischen dem Konjugat und den möglicherweise in dem Gewebe oder der biologischen Flüssigkeit vorhandenen Antikörpern erlauben, und den in vitro-Nachweis von möglicherweise gebildeten Antigen/Antikörper-Komplexen.

11. Verwendung eines Konjugats nach einem der Ansprüche 1 bis 4 in der Zubereitung eines Anti-Malaria-Impfstoffs.
